(19) Europäisches Patentamt
European Patent Office
Office européen des brevets

(11) **EP 2 064 542 B1**

(12) **EUROPEAN PATENT SPECIFICATION**

(45) Date of publication and mention
of the grant of the patent:
**17.08.2011 Bulletin 2011/33**

(21) Application number: **08742522.9**

(22) Date of filing: **03.04.2008**

(51) Int Cl.:
***G01N 33/487*** (2006.01)   ***G01N 33/543*** (2006.01)
***G01N 33/569*** (2006.01)

(86) International application number:
**PCT/US2008/004340**

(87) International publication number:
**WO 2008/130488 (30.10.2008 Gazette 2008/44)**

(54) **LIVE-CELL SIGNALS OF PATHOGEN INTRUSION AND METHODS THEREOF**

LEBENDZELLSIGNALE FÜR DAS EINDRINGEN VON KRANKHEITSERREGERN UND
ENTSPRECHENDE VERFAHREN

SIGNAUX DE L'INTRUSION DE PATHOGÈNES SUR UNE CELLULE VIVANTE ET PROCÉDÉS
ASSOCIÉS

(84) Designated Contracting States:
**CH DE FR GB LI**

(30) Priority: **19.04.2007 US 925274 P**

(43) Date of publication of application:
**03.06.2009 Bulletin 2009/23**

(73) Proprietor: **Corning Incorporated**
**Corning NY 14831 (US)**

(72) Inventors:
• **FANG, Ye**
**Painted Post, NY 14870 (US)**
• **LAHIRI, Joydeep**
**Painted Post, NY 14870 (US)**
• **VERRIER, Florence**
**Corning, NY 14830 (US)**

(74) Representative: **Oldroyd, Richard Duncan et al**
**Elkington and Fife LLP**
**Prospect House**
**8 Pembroke Road**
**Sevenoaks, Kent TN13 1XR (GB)**

(56) References cited:
**WO-A-2007/015878    US-A1- 2006 223 051**

• **FANG YE ET AL: "Resonant waveguide grating biosensor for living cell sensing." BIOPHYSICAL JOURNAL 1 SEP 2006, vol. 91, no. 5, 1 September 2006 (2006-09-01), pages 1925-1940, XP002488203 ISSN: 0006-3495**
• **FANG YE: "Label-free cell-based assays with optical biosensors in drug discovery." ASSAY AND DRUG DEVELOPMENT TECHNOLOGIES OCT 2006, vol. 4, no. 5, October 2006 (2006-10), pages 583-595, XP002488211 ISSN: 1540-658X**

**Description**

## BACKGROUND

The disclosure relates to optical biosensors, such as resonant waveguide grating (RWG) biosensors or surface plasmon resonance (SPR) biosensors, and more specifically to the use of such biosensors in live-cell sensing of pathogen intrusion and methods thereof.

US 2006/0223051 describes a system and method for using an optical label independent detection biosensor to screen compounds against a target G-protein coupled receptor (GPCR) within living cells based on the mass redistribution due to agonist-induced GPCR-activation.

## SUMMARY

The disclosure provides direct and indirect methods to detect a pathogen, such as a virus, and provides a measure of the pathogen's impact on a live-cell sample or a live-cell model.

## BRIEF DESCRIPTION OF THE DRAWINGS

Fig. 1 illustrates exemplary pathways that may be used by pathogens to commandeer normal cellular function and control, in embodiments of the disclosure.

Fig. 2 shows a schematic of exemplary signal events in adenoviral cell entry.

Fig. 3 shows a schematic of a *loci* for possible therapeutic intervention in the treatment of inflammation.

Figs. 4A and 4B show exemplary methods for detecting viral intervention in a label independent detection optical wave-guide grating biosensor system, in embodiments of the disclosure.

Figs. 5A and 5B, respectively, show exemplary biosensor measurements and results of an adenoviral infection mediated Gq signaling interference, .

Figs. 6A and 6B, respectively, show exemplary biosensor measurements and results of an adenoviral infection mediated $G_s$ signaling interference, .

Figs. 7A, 7B, and 7C show exemplary biosensor measurements of the effect of an adenoviral infection upon the response of A431 cells induced by epidermal growth factor (EGF) 32 nM,.

Figs. 8A and 8B show phosphoarray results for phosphorylation of 4 signaling proteins in infected or non infected A431 cells after stimulation,.

Fig. 9 shows a schematic of an example of signaling of cell migration, .

Fig. 10 shows a schematic of an example of G-protein-coupled-receptor, EGF receptor and focal adhesion signaling, .

Fig. 11 shows kinetic responses of HeLa cells to adenoviral infection, .

Fig. 12 shows modulation of the adenovirus-induced response in HeLa cells, .

Fig. 13 shows example results of dynamin inhibitory peptide (DIPC) inhibition of an adenoviral infection in HeLa cells.

## DETAILED DESCRIPTION

Various embodiments of the disclosure will be described in detail with reference to drawings, if any. Reference to various embodiments does not limit the scope of the invention, which is limited only by the scope of the claims attached hereto. Additionally, any examples set forth in this specification are not intended to be limiting and merely set forth some of the many possible embodiments for the claimed invention.

Definitions

"Assay," "assaying" or like terms refers to an analysis to determine, for example, the presence, absence, quantity, extent, kinetics, dynamics, or type of a cell's optical or bioimpedance response upon stimulation with an exogenous stimuli, such as a ligand candidate compound or a viral particle or a pathogen.

"Attach," "attachment," "adhere," "adhered," "adherent," "immobilized", or like terms generally refer to immobilizing or fixing, for example, a surface modifier substance, a compatibilizer, a cell, a ligand candidate compound, and like entities of the disclosure, to a surface, such as by physical absorption, chemical bonding, and like processes, or combinations thereof. Particularly, "cell attachment," "cell adhesion," or like terms refer to the interacting or binding of cells to a surface, such as by culturing, or interacting with cell anchoring materials, compatibilizer (e.g., fibronectin, collagen, lamin, gelatin, polylysine, etc.), or both.

"Adherent cells" refers to a cell or a cell line or a cell system, such as a prokaryotic or eukaryotic cell, that remains associated with, immobilized on, or in certain contact with the outer surface of a substrate. Such type of cells after culturing can withstand or survive washing and medium exchanging process, a process that is prerequisite to many cell-based assays. "Weakly adherent cells" refers to a cell or a cell line or a cell system, such as a prokaryotic or eukaryotic cell, which weakly interacts, or associates or contacts with the surface of a substrate during cell culture. However, these types of cells, for example, human embryonic kidney (HEK) cells, tend to dissociate easily from the surface of a substrate by physically disturbing approaches such as washing or medium exchange. "Suspension cells" refers to a cell or a cell line that is preferably cultured in a medium wherein the cells do not attach or adhere to the surface of a substrate during the culture. "Cell culture" or "cell culturing" refers to the process by which either prokaryotic or eukaryotic cells are grown under controlled conditions. "Cell culture" not only refers to the culturing of cells derived from multicellular eukaryotes, especially animal cells, but also the culturing of complex tissues and organs.

"Cell" or like term refers to a small usually microscopic mass of protoplasm bounded externally by a semipermeable membrane, optionally including one or more nuclei and various other organelles, capable alone or interacting with other like masses of performing all the fundamental functions of life, and forming the smallest structural unit of living matter capable of functioning independently including synthetic cell construct, cell model systems, and like artificial cellular systems.

"Cell system" or like term refers to a collection of more than one type of cells (or differentiated forms of a single type of cell), which interact with each other, thus performing a biological or physiological or pathophysiological function. Such cell system includes an organ, a tissue, a stem cell, a differentiated hepatocyte cell, or the like.

"Marker" or like term refers to a molecule, a biomolecule, or a biological that is able to modulate the activities of at least one cellular target (e.g., a $G_q$-coupled receptor, a $G_s$-coupled receptor, a $G_i$-coupled receptor, a $G_{12/13}$-coupled receptor, an ion channel, a receptor tyrosine kinase, a transporter, a sodium-proton exchanger, a nuclear receptor, a cellular kinase, a cellular protein, etc.), and thereby result in a reliably detectable biosensor output as measured by a biosensor. Depending on the class of the intended cellular target and its subsequent cellular event(s), a marker could be an activator, such as an agonist, a partial agonist, an inverse agonist, for example, for a GPCR or a receptor tyrosine kinase or an ion channel or a nuclear receptor or a cellular enzyme adenylate cyclase. The marker could also be an inhibitor for certain classes of cellular targets, for example, an inhibitor or a disruptor for actin filament, or microtuble.

"Detect" or like terms refer to an ability of the apparatus and methods of the disclosure to discover or sense a pathogen intrusion and to distinguish the sensed intrusion of a pathogen from an absence of a pathogen.

"Identify" or like terms refer to an ability of the apparatus and methods of the disclosure to not only recognize a pathogen's presence but to also classify the pathogen.

"intrusion" or like terms refer to a pathogen's ability to alter at least one of a cell's signal pathways. The intrusion event does not require physical entry of a pathogen or a component of the pathogen into the cell.

"Pathogen" or like terms refer to, for example, a virus, a bacterium, a prion, and like infectious entities, or combinations thereof.

"Therapeutic candidate compound," "therapeutic candidate," "prophylactic candidate," "prophylactic agent," "ligand candidate," or like terms refer to a molecule or material, naturally occurring or synthetic, which is of interest for its potential to interact with a cell attached to the biosensor or a pathogen. A therapeutic or prophylactic candidate can include, for example, a chemical compound, a biological molecule, a peptide, a protein, a biological sample, a drug candidate small molecule, a drug candidate biologic molecule, a drug candidate small molecule-biologic conjugate, and like materials or molecular entity, or combinations thereof, which can specifically bind to or interact with at least one of a cellular target or a pathogen target such as a protein, DNA, RNA, an ion, a lipid or like structure or component of a living cell or a pathogen.

"Biosensor" or like terms refers to a device for the detection of an analyte that combines a biological component with a physicochemical detector component. The biosensor typically consists of three parts: a biological component or element (such as tissue, microorganism, pathogen, cells, or combinations thereof), a detector element (works in a physicochemical way such as optical, piezoelectric, electrochemical, thermometric, or magnetic), and a transducer associated with both components. The biological component or element can be, for example, a living cell, a pathogen, or combinations thereof. In embodiments, an optical biosensor can comprise an optical transducer for converting a molecular recognition or molecular stimulation event in a living cell, a pathogen, or combinations thereof into a quantifiable signal.

Abbreviations, which are well known to one of ordinary skill in the art, may be used (e.g., "h" or "hr" for hour or hours, "g" or "gm" for gram(s), "mL" for milliliters, and "rt" for room temperature, "nm" for nanometers, and like abbreviations).

"Include," "includes," or like terms means including but not limited to.

"About" modifying, for example, the quantity of an ingredient in a composition, concentrations, volumes, process temperature, process time, yields, flow rates, pressures, and like values, and ranges thereof, employed in describing the embodiments of the disclosure, refers to variation in the numerical quantity that can occur, for example, through typical measuring and handling procedures used for making compounds, compositions, concentrates or use formulations; through inadvertent error in these procedures; through differences in the manufacture, source, or purity of starting materials or ingredients used to carry out the methods; and like considerations. The term "about" also encompasses amounts that differ due to aging of a composition or formulation with a particular initial concentration or mixture, and amounts that differ due to mixing or processing a composition or formulation with a particular initial concentration or mixture. Whether modified by the term "about" the claims appended hereto include equivalents to these quantities.

"Consisting essentially of" in embodiments refers, for example, to a surface composition, a method of making or using a surface composition, formulation, or composition on the surface of the biosensor, and articles, devices, or apparatus of the disclosure, and can include the components or steps listed in the claim, plus other components or steps that do not materially affect the basic and novel properties of the compositions, articles, apparatus, and methods of making and use of the disclosure, such as particular reactants, particular additives or ingredients, a particular agents, a particular cell or cell line, a particular surface modifier or condition, a particular ligand candidate, or like structure, material, or process variable selected. Items that may materially affect the basic properties of the components or steps of the disclosure or may impart undesirable characteristics to the present disclosure include, for example, decreased affinity of the cell for the biosensor surface, decreased affinity of the ligand candidate for a cell, decreased affinity of a pathogen for a cell, anomalous or contrary cell activity in response to a ligand candidate or like stimulus, and like characteristics.

The indefinite article "a" or "an" and its corresponding definite article "the" as used herein means at least one, or one or more, unless specified otherwise.

Specific and preferred values disclosed for components, ingredients, additives, cell types, pathogens, and like aspects, and ranges thereof, are for illustration only; they do not exclude other defined values or other values within defined ranges. The compositions, apparatus, and methods of the disclosure include those having any value or any combination of the values, specific values, more specific values, and preferred values described herein.

In embodiments the disclosure provides biosensors, such as resonant waveguide grating (RWG) biosensors or surface plasmon resonance (SPR) biosensors, and to methods for live-cell pathogen intrusion detection and diagnosis in, for example, viral infection of cellular systems. The disclosure also provides biosensor-based methods that can be used to identify anti-pathogen strategies and therapies, such as anti-viral therapeutic agents, such as remedial or prophylactic compounds, anti-inflammatory agents, and auto-immune agents.

**Direct Method**

The disclosure provides methods to directly monitor pathogen intrusion, such as viral infection, in host cell lines using, for example, Mass Redistribution Cell Assay Technology (MRCAT) with a Corning® Epic® biosensor system.

The disclosure provides an apparatus and method for the direct measurement of pathogen intrusion in a live-cell which can be useful in detecting, controlling, or avoiding the consequence of, for example, viral infection.

The disclosure provides a label-free method to a detect pathogen intrusion in a live-cell, the method comprising:

providing an optical biosensor having a live-cell immobilized on a surface of the optical biosensor;
contacting the immobilized cell on the surface of the biosensor with a pathogen; and
detecting a change in the cell's local mass or local mass density within the detection zone of the biosensor relative to the cell prior to pathogen contact.

**Indirect Method**

To further improve the sensitivity of the abovementioned direct method for monitoring pathogen intrusion, such as viral infection and events associated therewith, a second indirect approach was developed which is based upon a virus's propensity to hijack or commandeer one or more of a cell's signaling pathways.

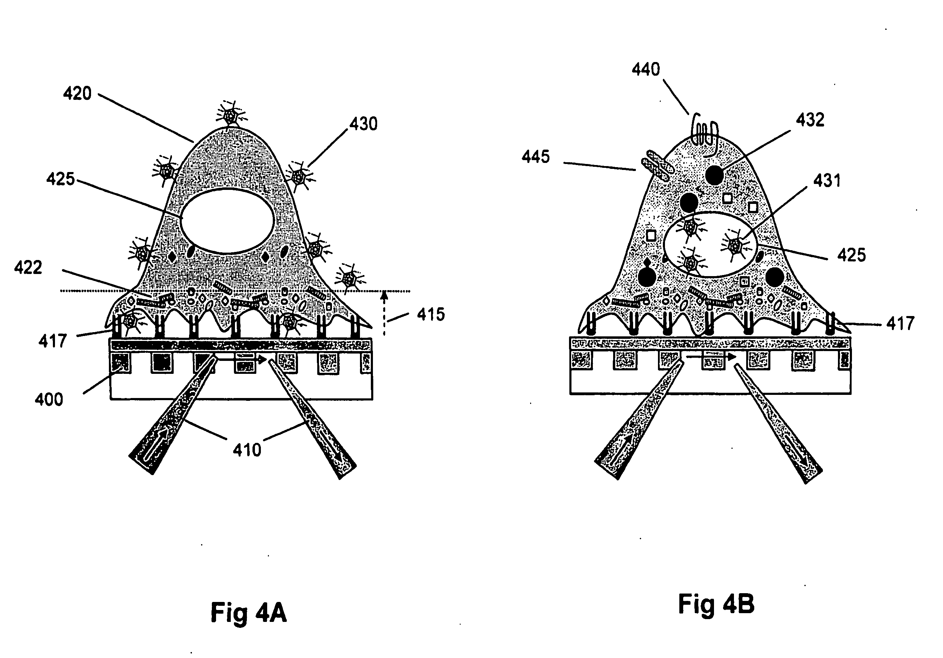

In embodiments, the indirect approach can comprise a panel of markers, each of which modulates at least one distinct cellular target, such as a receptor, which can subsequently trigger a change or a variation, such as activation, inhibition, and like changes, to one or more cell signal pathway(s), for example, GPCR signaling pathway, $Ca^{2+}$ pathway, mitogen-activated protein kinase (MAPK) pathway, adhesion pathway, cAMP pathway, AKT signaling pathway, apoptotic pathway, cell cycle pathway, receptor tyrosine kinase (RTK) signaling pathway, integrin signaling pathway, and like pathways, or combinations thereof. The impact of a viral infection on the marker-induced biosensor output signals can be used as an indicator and measure of the extent and type of intrusion, such as the mechanism(s) of viral infection as well as the cellular consequences of the viral infection (such as mentioned below and shown in Fig. 4).

The disclosure provides an apparatus and method for the indirect measurement of pathogen intrusion in a live-cell, which can also be useful in detecting, controlling, or avoiding the consequence of, for example, viral infection.

The disclosure provides a label-free method to detect a pathogen intrusion in a live-cell, the method comprising:

> providing an biosensor having a live-cell immobilized on a surface of the biosensor;
> contacting or exposing the immobilized cell on the surface of the biosensor with a pathogen;
> detecting a cell signaling or a cell-signal pathway perturbation in a panel of markers that modulate distinct cellular targets; and
> equating the extent of the perturbation with the extent of pathogen intrusion.

The disclosure provides a method for characterizing the effect of a pathogen on a cell, the method comprising:

> mapping a cell-signaling or cell-signal network profile resulting from exposure of an immobilized cell to a pathogen in accord with the preceding embodiment;
> comparing the mapped profile with a library of pathogen profiles; and
> identifying a profile from the library of pathogen profiles that corresponds to the mapped profile. The characterization of the effect of a pathogen on a cell can include, for example, identification of a pathogen responsible for the effect. Identifying a profile from the library of pathogen profiles can include, for example, selecting a library profile that is an exact match or a best match of the mapped profile. The method for characterizing the effect of a pathogen on a cell further comprise contacting the immobilized cell with a prophylactic candidate or remedial candidate before or after the step of mapping the cell signal network profile resulting from exposure of an immobilized cell to a pathogen.

For a given cell or cell system, a panel of markers, each of which or, for example, at least two or more can result in a reliable and detectable biosensor signal, can be predetermined and selected. For example, when optical biosensor such as RWG biosensor is used, in human epidermoid carcinoma A431 cells a panel of markers can be selected from the following group or groups:

An agonist or a partial agonist for endogenous GPCRs (e.g., bradykinin for bradykinin B2 receptor, epinephrine for β2 adrenergic receptor, adenosine for adenosine A2B receptor, thrombin or SFLLR-amide for protease activated receptor subtype 1, trypsin or SLIGKV-amide for protease activated receptor subtype 2, histamine for histamine H I receptor, adenosine triphosphate (ATP) for P2Y receptors, lysophosphatidic acid (LPA) for LPA receptors) (Fang, Y., et al., J. Pharmacol. Tox. Methods, 2007, 55, 314-322).

An agonist for endogenous receptor tyrosine kinase (e.g., epidermal growth factor (EGF) for EGFR) (Fang, Y., et al., Anal. Chem., 2005, 77, 5720-5725).

An ion channel opener for an endogenous ion channel (e.g., pinacidil for ATP-sensitive potassium ion channel).

An activator for a cellular enzyme (e.g., forskolin for adenylate cyclase).

A disrupting agent (e.g., cytochalasin D for actin filament, or nocodozale for microtubules).

An activator for integrin receptor (e.g., soluble fibronectin or its fragments).

A cell membrane disrupting agent (e.g., saponin to cause cell membrane leakage) (Fang, Y., et al., FEBS Lett., 2005, 579, 4175-4180).

An apoptotic inducer (e.g., $Ca^{2+}$ ionophore A23187 to trigger a $Ca^{2+}$ dependent cell apoptosis).

Since stimulation of the cells examined with each marker leads to a specific cellular event, a signaling pathway, or signaling network interactions, and each signaling pathway may involve distinct sets of cellular targets, the selected panel of markers will cover many, if not all, of the cellular signaling pathways in the given cell system. In contrast, each type of pathogen can alter or modulate a cell or cell system in a unique manner (i.e., a specific pathogen only selectively hijacks certain cellular targets). Therefore, the impact of pathogen intrusion on the biosensor output signals induced by the selected panel of markers produces a signature of the pathogen studied in the cell or cell system examined. Such mapping approach provides substantially greater sensitivity to pathogen intrusion detection compared to the abovementioned direct approach.

## Continuous or Hybrid Method

The disclosure provides a continuous or hybrid method to monitor the effect of pathogen intrusion in a live-cell, the method comprising:

providing a live-cell having a pathogen intrusion to a biosensor surface;
culturing the live-cell having the pathogen intrusion with the biosensor surface until a defined confluency is achieved; and
measuring the biosensor output during the cell culture and intrusion.

The continuous or hybrid method to monitor the effect of pathogen intrusion in a live-cell, can be modified to comprise:

providing a live-cell having a pathogen intrusion to a biosensor surface;
culturing the live-cell having the pathogen intrusion with the biosensor surface until a defined confluency is achieved; and
measuring the biosensor output for a predetermined and selected panel of markers.

In embodiments of the foregoing monitoring method, the biosensor can continuously monitor the course of the pathogen intrusion, the marker-induced cell-signal changes, or both, and can provide useful information regarding the effect of the pathogen intrusion on the state of the cell (e.g., cell growth, cell health, degree of cell adhesion, and like metrics).

In embodiments the disclosure provides methods of label-free or label-independent-detection (LID) optical biosensors, including SPR or RWG, to detect or identify pathogen intrusion in a live-cell, for example, a viral infection of live-cell such as in surface adherent live-cell cultures.

Using adenoviral infection as a model, we have demonstrated the following for the indirect marker-panel assay approach: 1) high sensitivity to viral infection detection having, for example, a desired sensitivity for diagnostics applications of from about 1 to about 100 viral particles per cell; and 2) the adenovirus infection hijacked the MAPK pathway, particularly adhesion pathways, but not $G_q$ pathway, at doses below about 1,000 viruses per cell.

Using such indirect and cell signaling mapping approach, for each virus, viral hijacking of cell signaling can be defined or determined, and then catalogued. Additionally, markers for multiple signaling pathways or networks can be determined and selected. Appropriate biosensor responses can be determined for use in, for example, viral detection and inflammatory drug discovery. The indirect method permits the detection of a virus in a sample and can enable the screening of modulators that may affect viral entry and the function of viral encoded cellular targets.

The disclosure provides advantaged label-free methods to detect pathogen intrusion, such as in viral infection. This method enables, for example, a rapid viral detection scheme without the use of amplification methods. The method permits the screening of, for example, candidate drug compounds that can block or "connect" the affected cellular physiology, for example, block viral infection partially or entirely, or block the function of viral encoded cellular target(s). In addition to viral assay applications, the methods of the disclosure can be used to screen drug candidate compounds or like materials that can potentially "correct" affected cellular physiology. The method of the disclosure can also provide tools useful in other therapeutic or diagnostic areas, such as in anti-inflammatory drug discovery or mapping inflammation cell-pathways.

Viruses use surprisingly diverse methods to hijack cell function, for example, signaling through G-protein-coupled receptors (GPCRs), and to harness the cell's own activated intracellular-signaling pathways. These methods ultimately function to ensure viral replication success and can often contribute to the virus's pathogenesis. A single virus may, for example, deploy a repertoire of these strategies to regulate key intracellular survival, proliferative, and chemotactic pathways. An understanding of the contributions of these biological or physiological or pathophysiological routes to viral pathogenesis can lead to development of effective target-specific therapeutic strategies against viral-induced diseases. Furthermore, understanding the mechanisms used by a virus to alter the cell signaling machinery can provide further insight into the mechanism by which autoimmune diseases develop. Additionally, understanding the role of inflammation in viral infection can lead to new therapeutic strategies that can ultimately enhance immune restoration and limit the formation of viral reservoirs in infected patients.

The methods of the disclosure provide high sensitivity over existing methods for diagnostic or study of viral infection. The sensitivity of most available detection methodologies used to demonstrate viral impact on cell signaling is, for example, from about 50 to about 1,000 particles/cell, such as in phosphorylation assays. In assay methods of the disclosure it was possible to detect a cell signaling perturbation using a viral concentration below, for example, about 1 viral particle/cell in the case of EGFR signaling.

**Biosensor-based cell signaling network mapping to detect pathogen intrusion**

*Theory of optical biosensor for whole cell sensing* - Beside its ability to monitor molecular interactions, the optical biosensor exploits an evanescent wave to detect ligand-induced alterations of a cell layer at or near the biosensor surface. The evanescent wave, which is an electromagnetic field created by the total internal reflection of guided light at a solution-surface interface, has a well-characterized short penetration depth or sensing volume typically about 200 nm. Because a living cell has comparatively large dimensions, the optical biosensor sensor is considered to be a nonconventional three-layer system comprising: a substrate; a waveguide film in which a grating structure is embedded; and a cell layer. Therefore, a ligand-induced change in the effective refractive index (i.e., the detected signal) is, to a first order, directly proportional to the change in the refractive index of the bottom portion of cell layer nearest the waveguide film according to equation (1):

$$\Delta N = S(C)\Delta n_c \qquad (1)$$

where S(C) is the sensitivity to the cell layer, and $\Delta n_c$ is the ligand-induced change in local refractive index of the cell layer sensed by the biosensor.

The $\Delta n_c$ value is directly a function of change in local concentrations of cellular targets or molecular assemblies within the sensing volume. This is because of a well-known physical property of cells - the refractive index of a given volume within a cell is largely determined by the concentrations of bio-molecules, mainly proteins, which is also the basis for contrast in light microscopic images of cells. Considering the exponentially decaying nature of the evanescent wave, a detected signal is a sum of mass redistribution occurring at distinct distances away from the sensor surface, each with uneven contribution to the overall response. Taking the weighed factor $\exp(-zi/\Delta Z_c)$ into account, the detected signal occurring perpendicular to the sensor surface is governed by:

$$\Delta N = S(N)\alpha d \sum_i \Delta C_i \left[ e^{\frac{-z_i}{\Delta z_c}} - e^{\frac{-z_{i+1}}{\Delta z_c}} \right] \qquad (2)$$

where $\Delta Z_c$ is the penetration depth into the cell layer, a is the specific refraction increment (about 0.18/mL/g for proteins), $z_i$ is the distance where the mass redistribution occurs, and d is an imaginary thickness of a slice within the cell layer. Here the cell layer is divided into an equal-spaced slice in the vertical direction.

Our analysis suggested that a resonant waveguide grating (RWG) biosensor can detect ligand-induced dynamic mass redistribution (DMR) within the bottom portion of an adherent cell layer. Our pharmacological studies suggested that the DMR signal can serve as a novel physiological readout for monitoring receptor activation, and for examining ligand pharmacology. Our biochemical studies supported the hypothesis that the DMR signal is an integrated response that consists of contributions from many cellular events induced by the ligand, thus providing an alternative means to study cell systems biology.

*Theory of electrical biosensor for whole cell sensing* - Electrical biosensors consist of a substrate (e.g., plastic), an electrode, and a cell layer. In this electrical detection method, cells are cultured on small gold electrodes arrayed onto a substrate, and the system's electrical impedance is followed with time. The impedance is a measure of changes in the electrical conductivity of the cell layer. Typically, a small constant voltage at a fixed frequency or varied frequencies is applied to the electrode or electrode array, and the electrical current through the circuit is monitored over time. The ligand-induced change in electrical current provides a measure of cell response. The application of impedance measurements for whole cell sensing was first realized in 1984 (Giaever, I.; Keese, C.R. Proc. Natl. Acad Sci. U.S.A., 1984, 81, 3761). Since then, impedance-based measurements have been applied to study a wide range of cellular events, including cell adhesion and spreading, cell micromotion, cell morphological changes, and cell death. Classical impedance systems suffer from high assay variability due to use of a small detection electrode and a large reference electrode. To overcome this variability, the latest generation of systems, such as CellKey system (MDS Sciex, South San Francisco, CA) and RT-CES (ACEA Biosciences Inc., San Diego, CA), utilize an integrated circuit having a microelectrode array.

In a typical impedance-based cell assay, cells are brought into contact with a gold electrode arrayed on the bottom of culture wells. The total impedance of the sensor system is determined primarily by the ion environment surrounding the biosensor. Under application of an electrical field, the ions undergo field-directed movement and concentration gradient-driven diffusion. For whole cell sensing, the total electrical impedance has four components: the resistance of the electrolyte solution, the impedance of the cell, the impedance at the electrode/solution interface, and the impedance at the electrode/cell interface. In addition, the impedance of a cell comprises two components - the resistance and the reactance. The conductive characteristics of cellular ionic strength provide the resistive component, whereas the cell membranes, acting as imperfect capacitors, contribute a frequency-dependent reactive component. Thus, the total impedance is a function of many factors, including cell viability, cell confluency, cell numbers, cell morphology, degree of cell adhesion, ionic environment, the water content within the cells, and the detection frequency.

In the RT-CES system, a percentage of this small voltage applied is coupled into the cell interior. Such signals applied to cells are believed to be much smaller than the resting membrane potential of a typical mammalian cell and thus present minimal or no disturbance to cell function. The RT-CES system measures these changes in impedance and displays it as a parameter called the cell index. The cell index is calculated according to the formula (Solly, K.; Wang, X.; Xu, X.; Strulovici, B.; Zheng, W. Assays Drug Dev. Technol. 2004, 2, 363):

$$CI = \max_{i=1,...,N}\left(\frac{R_{cell}(f_i)}{R_0(f_i)} - 1\right) \qquad (3)$$

where N is the number of frequency points at which the impedance is measured (e.g., N=3 for 10 kHz, 25 kHz, and 50 kHz), and $R_0(f)$ and $R_{cell}(f)$ are the frequency electrode resistance without cells or with cells present in the wells, respectively.

In the CellKey system, a change in sensor system's impedance is attributed to a change in complex impedance (delta Z or dZ) of a cell layer that occurs in response to receptor stimulation (Verdonk, E.; Johnson, K.; McGuinness, R.; Leung, G.; Chen, Y.-W.; Tang, H.R.; Michelotti, J.M.; Liu, V.F. Assays Drug Dev. Technol., 2006, 4, 609). At low frequencies, the small voltage applied induces extracellular currents (iec) that pass around individual cells in the layer. However, the conduction currents through cell membrane due to ion channels may also be important at low measurement frequencies. At high frequencies, they induce trans-cellular currents (itc) that penetrate the cellular membrane. The ratio of the applied voltage to the measured current for each well is its impedance (Z) as described by Ohm's law.

When cells are exposed to a stimulus, such as a receptor ligand, signal transduction events are activated that lead to complex cellular events such as modulation of the actin cytoskeleton that cause changes in cell adherence, cell shape and volume, and cell-to-cell interaction. These cellular changes individually or collectively affect the flow of extracellular and trans-cellular current, and therefore, affect the magnitude and characteristics of the measured impedance. Similar to the optical biosensor, these electrical biosensors also enable the measurement of an integrated cellular response, related to the bio-impedance, mediated by the activation of a cellular receptor upon stimulation.

*Biosensor for systems cell biology* - Three important aspects that can qualify the suitability of a given approach for systems biology application include, for example, the ability to multiplex, the ability to accomplish a multi-parameter analysis, and the ability to obtain quantitative system-response profiles. The biosensor-based cell assays of the disclosure are capable of multiplexing, at least in two aspects. First, the activation of a same-class of targets (e.g., $G_q$-coupled receptors) in a given cell line leads to almost identical optical signatures, which suggests that multiple targets within the same family can be assayed at the same time. For example, A431 cells endogenously express bradykinin B2 receptor, P2Y receptors, and protease activated receptors (PARs). Upon stimulation with bradykinin, ATP, or thrombin, quiescent A431 cells respond with similar $G_q$-type optical signatures. Second, since some of the signaling components play important roles in an agonist-induced DMR signal mediated through the agonist's cognate target, multiple targets within the same signaling pathway can also be assayed at the same time. For example, the EGF-induced DMR in A431 can be used to profile the compounds that target one of its downstream targets such as MEK. MEK is a dual-specificity kinase that phosphorylates the tyrosine and threonine residues on ERKs 1 and 2 required for activation. Two related genes encode MEK1 and MEK2 which differ in their binding to ERKs and, possibly, in their activation profiles.

Optical biosensors offer multiple parameters to analyze the ligand-induced DMR responses. These parameters include the shift in angle or wavelength of the reflected light, that is, the interrogated light, which is sensitive to the vertical mass redistribution, and the parameters defining the shape of the resonant peak (e.g., intensity, peak area, and the peak-width-at-half-maximum (PWHM)) which parameters are mostly sensitive to the lateral mass redistribution. The combination of these parameters can further provide detailed information on the action of ligands in the cells examined. Alternatively, since the biosensor is non-invasive, the biosensor-based cell assays can be easily integrated with other technologies, such as mass spectroscopy and fluorescence imaging. These other technologies can corroborate the measured action of compounds or ligands in cells.

The DMR signal mediated through a particular target is an integrated and quantifiable signal that is a sum of contributions from mass redistribution occurring at different distances away from the sensor surface. Because of the complex nature of cell signaling, the activation of distinct cell signaling mediated through different targets might result in similar overall DMR signal. Since an ensemble of available targets and inhibitors for a signaling process may be *a priori* known, it is possible to determine the cell signaling activated through the target, based on the analysis of the modulation profiles of certain inhibitors on the ligand-induced DMR signal. The effect of an inhibitor on the optical responses (e.g., overall dynamics, kinetics, and amplitude of the response) is an indication of the role of the inhibitor-targeted biomolecule in the signaling. Therefore, the DMR response can be treated as a unique readout for systems biology study of living cells. We have applied the DMR signal of quiescent A431 cells induced by epidermal growth factor (EGF) to map the signaling pathways and network interaction of epidermal growth factor receptor (EGFR), and to study the cellular functions of cholesterol. Analysis of the modulation of the EGF-induced DMR signal by various known modulators provided links of various targets to distinct steps in the cellular responses, which links the EGF-induced DMR response of quiescent A431 cells mainly to the Ras/mitogen-activated protein (MAP) kinase pathway, which pathway primarily proceeds through MEK and leads to cell detachment.

*Optical biosensors for cell signaling network mapping* - The disclosure provides an alternative method to detect pathogen intrusion of a cell, such as the viral infection in a cell system. The method is based on systematic mapping of distinct cell signaling and its network interaction using the biosensor. In a given cell system, there are a great number of cellular targets endogenously expressed; the activation of each target leads to a cell signaling event. Some of these cell signaling can be assayed by the biosensor when there is significant dynamic redistribution of cellular matter within the detection zone of the biosensor. Using a panel of markers that activate distinct cellular targets, the biosensor enables the mapping of a great number of cell signaling pathways and their interactions. Based on the impact of viral infection on the marker-induced DMR signals, one can determine which signaling pathways or cascades, are altered by the virus, which can serve as an indirect measure of the pathogen intrusion. For different types of viruses, the pattern of such alteration may differ, thus the distinctive alteration patterns can be used for viral identification. Alternatively or additionally, the effect of a compound on the alteration pattern can be used to screen drugs that effect the viral infection.

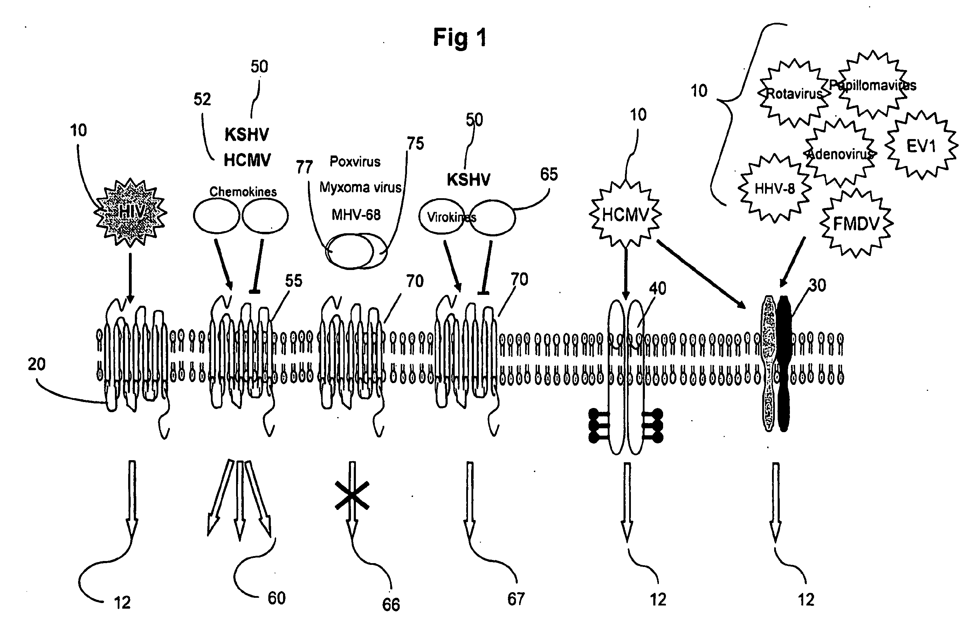

*Viral infection and cell signaling hijacking* - Viruses depend on the host cell's infrastructure for replication of their own genetic material and for production of their own capsid and envelope proteins. To reach the site of replication, a viral particle must bind to the host cell, penetrate the cellular membrane to enter the cytosol, and often also enter the nuclear envelope to allow replication of viral DNA. Therefore, viruses have evolved remarkable mechanisms to exploit every possible cellular system that might aid them in this path. Furthermore, many viruses do not kill the cell upon infection, but instead use the host's cellular machinery for efficiently propagating between cells and tissues. Referring to the Figures, Fig. 1 shows a schematic that illustrates example modalities of pathogen intrusion upon a cell's intracellular signaling networks, such as a viral pathogen hijacking one or more of the cell's intracellular signaling networks. Many viruses use, for example, cellular GPCR 20, Integrin 30, or EGFR 40 as coreceptors for facilitating viral entry 12, that is, direct interaction of viruses with these cellular receptors. Viruses as HCMV 50 or KSHV 52 might encode their own GPCRs 55, which often constitutively signal to a network of intracellular cascades 60. Virally encoded chemokines 65 (virokines) might also function as agonists 66 or antagonists 67 of cellular or viral GPCRs 70. Virally encoded chemokines-binding proteins (CKBPs) 75 bind to and sequester cellular chemokines 77; to prevent the activation of cellular GPCRs by endogenous chemokines.

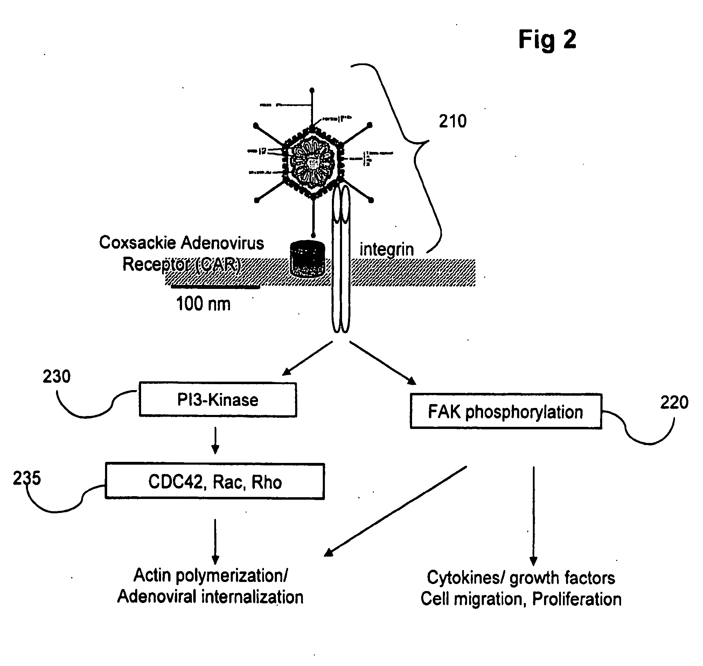

*Cell Signaling hijacking during viral entry* - Many viruses make use of the cell's signaling pathways during entry (Fig. 1 and Fig.2). To prepare a cell for the invasion, virus particles trigger events as soon as they bind to the plasma membrane. This generally involves the binding to and the cross-linking of cell-surface molecules such as glycosphingolipids, receptor tyrosine kinases, and integrins. This was first recognized for adenovirus, which uses Coxsackie Adenovirus Receptor (CAR) as a primary receptor and integrins as a co-receptor. Upon binding to its cellular receptors, adenovirus induces signaling cascades through phosphatidylinositol-3-OH kinase (PI3K) and Rac1, a small GTPase of the Rho family resulting in the polymerization of actin and clathrin-mediated endocytosis of the virus (Figs 1 and 2). Adenovirus induces this signaling for two reasons. First, the signaling leads to sequestration of the integrin-bound virus particle into clathrin-coated pits, which are subsequently internalized. Secondly, the actin cytoskeleton is reorganized to form membrane ruffles and macropinocytosis is increased. Activation of many different signaling pathways has since been described with the involvement of a variety of factors, including serine/threonine, tyrosine, and PI kinases, phosphatases, and a variety of small GTPases (including Arf, Rab and Rho family members).

Viruses use signaling activities to induce changes in the cell that promote viral entry and early cytoplasmic events, as well as to optimize later processes in the replication cycle. Initially a virus needs to make its presence on the cell surface known so that the cell can launch an endocytic response that results in viral entry. The "virus-present" signals can be generated in several ways. A virus may directly activate cellular signaling molecules by using them as receptors. Alternatively, virus may induce cell signaling by clustering specific cell-surface proteins or lipids. For example, a number of viruses use glycosyl-phosphatidylinositol (GPI)-anchored proteins and gangliosides, which are only associated with the outer leaflet of the plasma membrane, as their receptors. That this often leads to activation of tyrosine kinases on the cytosolic side may be related to the fact that the GPI-anchored proteins and gangliosides become lipid-raft associated when clustered. Accordingly, there is increasing evidence that many viruses associate with lipid rafts to initiate intracellular signaling.

Many other examples illustrate the cell signaling induction during the viral entry. HCMV (human cytomegalovirus) is a herpes virus that is known to activate several signaling pathways including phosphatidylinositol-3-OH kinase (PI3K), G-protein, and mitogen-activated protein kinase cascades. Recently, the epidermal growth factor receptor (EGFR) was identified as a cellular receptor of HCMV. Interestingly, echovirus 1, a virus that utilizes a different combination of integrins as a receptor ($\alpha 2\beta 1$), appears to activate other downstream events. Simian vacuolating virus (SV40) binds to glycosphingolipids and induces a signaling cascade that results in the tyrosine phosphorylation of proteins that localize to caveolae.

In many cases, virus-induced signaling leads to dynamic changes in the actin cytoskeleton. This might have several purposes. One is to increase or to activate endocytic activity. For instance, after binding to the cell surface, SV40 stimulates breakdown of both actin stress fibres and the cortical actin cytoskeleton to activate endocytosis of virus-loaded caveolae. Another purpose is to bring cell surface-bound virus particles to sites of high endocytic activity. Enveloped viruses, fusing directly with the plasma membrane, need to overcome the cortical actin barrier to efficiently infect the cell. Herpes Simplex virus HSV-1 probably tackles this problem by activation of $Ca^{2+}$ signaling pathways, which are known to induce cortical actin depolymerization and destabilization of focal adhesions. Actin rearrangements might also aid in the efficient spread of progeny virus particles, as observed for vaccinia virus (VV).

Actin rearrangements contribute to virus particle internalization, in most cases, by endocytosis. Ironically, although virus endocytosis might have been intended as a cellular defense mechanism aimed at destruction of the particle within lysosomes, many enveloped viruses hijack or take control of the process as the low pH in early and late endosomes provides a convenient cue for the virus to initiate membrane penetration. As a result, the virus takes a convenient ride into the cell and escapes exposure to degradative lysosomes.

A favored model for viral entry involves the binding of glycoprotein gp120 of Human Immunodeficiency Virus type I (HIV-1) to cluster of differentiation 4 (CD4) and subsequently to C-C chemokine receptor 5 (CCR5) or C-X chemokine receptor 4 (CXCR4) that promotes fusion between the viral and host membranes. The gp120 of HIV activates the extracellular signal-regulated kinase (ERK), Jun N-terminal kinase (JNK), and p38 pathway by engaging CD4 independently of CXCR4 or CCR5. The activation of these MAPKs can have several important consequences. ERK, p38, and JNK can affect the proliferative capacity of infected cells and facilitate HIV-1 replication through Activator Protein-1 (AP-1) and nuclear-factor (NF)-κB-mediated pathways that can enhance the expression of viral genes. The activation of these MAPKs might also promote the production and release of numerous cytokines, which can function in an autocrine or paracrine manner to regulate viral replication. Therefore, in addition to functioning as an essential HIV-co-receptor, the ability to signal to intracellular MAPKs by CCR5 might facilitate HIV-1 infection. Signaling pathways that are activated by CCR5 and CXCR4 receptors might also facilitate the propagation of HIV by promoting the recruitment of host cells to the site of infection. Similarly, gp120 binding to CXCR4 or CCR5 activates cytoplasmic tyrosine kinase PYK2 and focal adhesion kinase (FAK) independently of CD4. In this regard, HIV-1 can elicit changes in the activation and distribution of components of focal adhesion complexes. This could thereby enhance the recruitment of T cells and macrophages to the sites of viral production, and so favor viral spreading and dissemination. These cytoskeletal rearrangements could also regulate the post-entry steps of HIV infection, for example, by facilitating viral translocation to the nucleus. Finally, binding of the HIV-1 envelope to CD4-chemokine-receptor complexes might initiate a signaling pattern that is distinct from that induced by activation of either receptor by its natural cellular agonist.

*Cell Signaling Hijacking associated with viral encoded proteins* - Growing evidence indicates that infectious agents can be potent initial triggers, subverting and exploiting host cell signaling pathways. This role is exemplified by the association of parvovirus B19 (B19) with human autoimmune disease. Infection with this common virus exhibits striking similarities with systemic autoimmune diseases, and can be associated with elevated serum autoantibody titers. The B19 virus produces proline-rich, 11-kDa proteins that have been implicated in modulation of host signaling cascades involved in virulence and pathogenesis. Additionally, B19 produces a non-structural protein (NS1) which is involved in DNA replication, cell cycle arrest and initiation of apoptotic damage, particularly in erythroid cells. It is even more remarkable that NS1 functions as a trans-acting transcription activator for the interleukin-6 (IL6) promoter, upregulating IL6 expression in host cells. Hence, B19 infection may play a pivotal role in triggering inflammatory disorders. By promoting apoptotic damage and trans-activating pro-inflammatory cytokine promoters, B19 may upset the delicate balance between cell survival and apoptosis, and may contribute to immune deregulation.

The human T lymphotropic virus type 1 (HTLV-1) infects an estimated 15 to 20 million people worldwide. In about five percent of them, the infection will lead to adult T cell leukemia or lymphoma (ATLL). ATLL is an aggressive disease characterized by a long latent period and the proliferation of T lymphocytes. While the mechanisms involved are incompletely understood, viral proteins such as Tax and p12 may play a central role in these processes. p12 alters the activity of a variety of genes linked to chemical pathways that control cell signaling, proliferation, and death. The role of Tax in the deregulation of selected cellular-signaling pathways has been demonstrated. Specifically, this has focused on the influence and interaction of Tax with the AP-1 and NF-AT transcription factors, PDZ domain-containing proteins, Rho-GTPases, and the Janus kinase/signal transducer and activator of transcription and transforming growth factor-beta-signaling pathways.

A main feature of HIV infection is the expression of several pro-inflammatory cytokines. Moreover, several HIV proteins such as Nef, Tat, and Vpr hijack pro-inflammatory cytokine signaling, further suggesting the potential importance of inflammation in HIV pathogenesis. In vivo chronic inflammatory conditions have been correlated to increased levels of viremia and accelerated disease progression. This finding suggests inflammation may play a crucial role in both immune suppression and the formation of viral reservoirs during HIV infection.

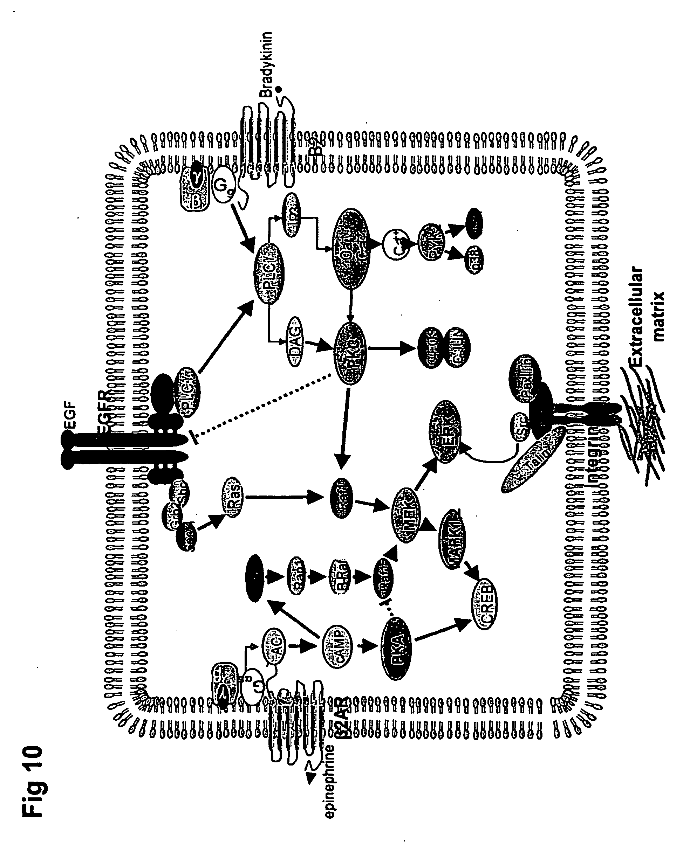

*Virus hijacking GPCR signaling networks* - G-protein-coupled receptors (GPCRs) constitute of the largest group of cell-surface proteins that are involved in signal transduction. GPCRs participate in a wide variety of physiological functions, including neurotransmission, exocytosis, angiogenesis, and like functions. GPCRs are also involved in a number of human diseases, which is reflected by the fact that GPCRs are the target (directly or indirectly) of about 50 to about 60% of all present therapeutic agents. The diversity of the biological responses that are elicited by GPCRs probably relies on the integration of the functional activity of an intricate network of intracellular signaling pathways, which include second-messenger-generating systems, small GTPases of the Ras and Rho families and their targets, and members of the mitogen-activated protein kinase (MAPK) family of serine/threonine kinases (as exemplified in Fig. 10). Given the versatility of GPCR signaling and its wide involvement in physiological processes, it is not surprising that viruses have evolved to exploit these receptors to their advantage (as exemplified in Fig. 1). This might be to recognize and infect target cells, or to harness their signaling in order to redirect normal cellular programs to evade immune-detection or to carry out the replicative needs of the virus. Indeed, a particular group of GPCRs that function as receptors for chemokines (as exemplified in Fig. 1) has been implicated in a wide range of virally induced diseases. Within the last decade, the key role of chemokine receptors in the pathogenesis of HIV has heightened awareness of their crucial function in viral pathogenesis. Likewise, the identification of the Kaposi's-sarcoma-associated herpesvirus (KSHV) GPCR as a viral gene that could be responsible for the initiation of Kaposi's sarcoma has further drawn attention to the importance of virally encoded GPCRs in human disease. Other strategies are used by viruses to hijack cellular GPCRs and exploit their activated intracellular signaling pathways. Such strategies include, for example, the modulation of the expression and function of cellular GPCRs and the expression of virally encoded ligands (virokines) or ligand-binding/sequestering proteins (as exemplified in Fig. 1). All of these strategies ultimately function to facilitate the propagation of the virus and thereby contribute, in many cases, to viral pathogenesis.

*Viral GPCRs* - The herpesvirus family of DNA viruses includes eight human pathogens. In spite of their divergent viral genomes and the distinct resulting conditions, many members of the herpesvirus family share a common strategy that ensures their replicative success: hijacking GPCR function from their cellular host. Emerging evidence suggests that these virally encoded GPCRs and their regulated signaling pathways have an essential role in viral pathogenesis and may represent new targets for therapeutic intervention in virally induced diseases.

The recent identification of Kaposi's-sarcoma-associated herpesvirus (KSHV) as the viral etiologic agent of Kaposi's sarcoma has renewed interest in the pathogenesis of this enigmatic disease. Kaposi's sarcoma is the most frequent type of tumor that occurs in HIV infected patients and remains a significant cause of death among the world's population of acquired immune deficiency syndrome (AIDS) sufferers. Kaposi's sarcoma lesions contain proliferating tumor cells, infiltrating inflammatory cells, extravasated erythrocytes, and abundant neovascular spaces. The KSHV genome encodes several candidate oncogenes. Among them, a virally encoded GPCR (KSHV GPCR) is unique in that it is both transforming and pro-angiogenic. The KSHV GPCR is highly related to the CXC family of chemokine receptors. KSHV also encodes a chemokine ligand, vMIP-II (discussed further below), that inhibits signaling by KSHV GPCR, to provide this virus with another control or feedback mechanism to modulate KSHV-GPCR activity. Compelling evidence now supports an essential role for KSHV GPCR in promoting tumor formation. KSHV GPCR potently stimulates the PI3K-AKT/PKB pathway in endothelial cells, which protects them from apoptosis. Therefore, KSHV GPCR may use this pathway to promote the survival of KSHV infected endothelial cells. Interestingly, KSHV GPCR can also activate AKT/PKB in an autocrine manner by upregulating the expression of the vascular endothelial growth factor (VEGF) receptor KDR2 and by promoting the concomitant release of VEGF, which then signals through the VEGF receptor to activate AKT/PKB. KSHV-GPCR-expressing endothelial cells can also induce AKT/PKB activity in neighboring endothelial cells *in* vivo through the release of VEGF and chemokines by a paracrine mechanism. KSHV-GPCR mediated oncogenesis therefore probably results from the interplay between direct and autocrine/paracrine cell transformation, and AKT/PKB may represent a point of convergence of both mechanisms. The transforming, pro-survival, and angiogenic effects of KSHV GPCR are also highly dependent on its ability to stimulate MAPKs, and consequently, the activity of transcription factors that are regulated by these kinases. KSHV GPCR can also activate the AP-1 and NF-κB transcription factors, which stimulate the expression of pro-inflammatory cytokines such as IL-1β, IL-2, IL-4, IL-6, tumor necrosis factor α(TNPα), CCL3/MIP-1, and IL-8/CXCL8, as well as basic fibroblast growth factor, all of which are important mediators in Kaposi's sarcoma.

Human cytomegalovirus (HCMV) is a widespread herpesvirus, as reflected by the presence of antibodies against HCMV proteins in 50-95% of the population. Although asymptomatic or subclinical in healthy populations, it can cause severe manifestations in immuno-compromised individuals, and remains the leading cause of congenital viral infection in humans, with an incidence as high as 0.2-2.2% of live births. HCMV is also detected in arterial tissues from individuals that are suffering from severe atherosclerosis, where it may participate in the transformation of arterial smooth-muscle cells (SMCs) and therefore lead to SMC focal proliferation, which is a hallmark of atherosclerotic disease. Among the HCMV-encoded proteins, four GPCRs, US27, US28, UL33 and UL78, stand out as likely candidates for involvement in HCMV-induced pathogenesis. US28 shows high homology to the CCR1 and CCR2 chemokine receptors. Because of its high affinity for many chemokines, US28 can sequester these cytokines, which facilitates immune evasion at sites of infection and contributes to the latent presence of the virus. This GPCR also promotes the migration of infected cells towards CC-chemokine-secreting tissues, which assists virus dissemination. US28 and its constitutive activity appear to be necessary for CMV to elevate the turnover of phosphatidylinositol in infected cells, which may promote migration. Therefore, US28-induced SMC migration, which involves activation of the tyrosine kinases SRC and FAK, may provide a molecular basis for the acceleration of vascular disease by HCMV, including the development of atherosclerosis. US28 also activates NF-κB through $\alpha\alpha$ dimers that are released from Gq/11, and activates CREB through p38, which indicates that US28 may regulate various transcription factors through G-protein-initiated signaling routes that control MAPKs. UL33 may not bind chemokines, but it can activate several signaling components in a ligand-independent manner, including phospholipase C (PLC) through Gq/11, and partially through Gi/o. In addition, UL33 constitutively modulates CRE (cyclic-AMP response element)-mediated transcription by coupling to Gi/o and Gs, and controls the intracellular levels of cAMP, as well as signaling through the Rho-p38 pathway. Activation of CRE, in turn, may promote the expression of molecules that stimulate cell growth, such as cyclin D. It is tempting to speculate that HCMV US28 and UL33 may contribute to the observed transformation of SMCs in atherosclerosis by activating ERK- and p38-dependent proliferative signaling pathways.

HHV6 can cause *exanthema subitum* in infants, other febrile illnesses in young children, and an infectious mononucleosis-like illness in adults. HHV6 encodes two GPCRs, which are known as open reading frames U12 and U51. HHV6 U12 shows the highest homology with CCR3 and is a promiscuous high-affinity CC-chemokine receptor, which increases intracellular $Ca^{2+}$ concentrations through a pertussis-toxin-insensitive pathway. The HHV6 U51 chemokine receptor is quite different to other virally encoded GPCRs, as it binds chemokines such as CCL5/RANTES, but its primary sequence is closer to that of the opioid receptors than to chemokine receptors. However, the effects of U51 and its intervening signaling pathways have not been fully explored.

Epstein-Barr virus (EBV/HHV4). Although EBV is the only α-herpesvirus that does not encode a chemokine receptor, viral infection of B cells leads to the up-regulated expression of endogenous cellular GPCR chemokine receptors including CCR6, CCR7, and CCR10. Activation of CCR7 by its endogenous ligands can then stimulate the AKT/PKB and ERK signaling pathways, in addition to activating JAK-STAT signaling. Up-regulated expression of CCR7 may therefore help to promote survival and proliferation of EBV-infected cells. Furthermore, CCR7 has also been implicated in lymphocyte migration through the activation of several Rho GTPases, including Rho, Rac, and Cdc42. CCR7 might therefore further participate in promoting the migration of infected cells, thereby facilitating viral spread. Nonetheless, the potential contribution of up-regulation of cellular GPCRs in herpesviral diseases warrants further investigation.

Poxviruses are a family of large, double-stranded-DNA viruses that include the smallpox virus (vareola), which causes a severe disease that has been virtually eliminated by vaccination. Similar to the case for herpesviruses, during the course of evolution, poxviruses have acquired genes that evade or prevent the host's immune response. These genes include those for numerous virally encoded cytokines and chemokine binding proteins. Recent sequence analyses of poxvirus genomes have also revealed the presence of putative viral GPCRs. Among them, the yaba-like disease (YLD)-virus protein 7L, which is highly related to CCR8, is the first example for which binding to a chemokine, CCL1, was demonstrated. The result of such binding is the activation of ERK. Whether other open reading frames, that are found in poxviruses, encode functional GPCRs warrant further exploration, as does their biological role in viral infection.

*Virally encoded chemokines* - In addition to encoding their own (pirated) receptors, several DNA viruses have evolved to encode an extensive repertoire of secreted proteins that bind to receptors on host cells, that can induce or inhibit intracellular signaling pathways (as exemplified in Fig. 1). Like virally encoded GPCRs, viral cytokines (or virokines) were probably hijacked from their cellular host, and therefore share many similar structural and functional features with the host proteins. However, owing to the selection pressure to limit the size of viral genomes, virokines have evolved to become smaller and more potent. Most virokines defend viruses against the aggressive assault of the host immune cells. Indeed, targets of virokines include the interferon (IFN) system, TNFs, various interleukins, the Complement system, and antigen presentation by the Major Histocompatibility Complex (MHC). Not surprisingly, GPCRs represent a significant target for disruption or exploitation by virokines. Virokines either help the virus to evade immune detection or they cause the recruitment of leukocytes to increase the pool of new host cells, which thereby facilitates viral dissemination.

KSHV encodes three related viral MIP (vMIP) genes, vMIP-I, vMIP-II and vMIP-III, which have significant protein-sequence similarity to CC chemokines, but are more closely related to each other than to the cellular chemokines. vMIP-1 has a restricted binding profile, and specifically interacts with CCR8, which is the sole receptor for the human CC chemokine CCL1. vMIP-I induces the expression of VEGF in PEL cells, and can rescue them from chemically induced apoptosis. The anti-apoptotic effects of vMIP-I seem to involve the activation of the ERK signaling pathway. In contrast to vM7P-I, which is an agonist for CCR8, vMIP-II is antagonistic for 10 chemokine receptors, which cover all four classes: XCR, CCR, CXCR and CX3CR. The wide spectrum of human CC and CXC chemokine receptors to which vMIP-II binds includes: the CCR3 receptor (which is involved in the trafficking of eosinophils and TH2 lymphocytes); the CXCR4 and CCR5 receptors; the CCR8 receptor; and the KSHV-encoded KSHV GPCR. So vMIP-II can: activate and chemoattract human eosinophils and TH2 lymphocytes; prevent cell entry of HIV; inhibit chemokine-mediated $Ca^{2+}$ mobilization, and limit the signaling ability of the KSHV GPCR. vMIP-III binds to and activates CCR4, thereby selectively chemo-attracting TH2 cells. All three vMIPs are also pro-angiogenic, and probably contribute to the neo-vascular phenotype of Kaposi's sarcoma lesions.

HCMV encodes two proteins, vCXC-1 and vCXC-2, that have sequence similarity to the CXC chemokines. vCXC-1 is a 117-amino-acid secreted glycoprotein that induces $Ca^{2+}$ mobilization, chemotaxis, and degranulation of neutrophils. High-affinity agonistic vCXC-1 binding is mediated through CXCR2, but not CXCR1. Stimulation of CXCR2 by its natural agonist, IL-8/CXCL8, activates a network of intracellular signaling pathways, which have been hijacked by other herpesviruses. Which (if any) of these biochemical routes are regulated by vCXC-1 (and vCXC-2) is under investigation.

HHV6 encodes a virokine, vCCL4, the expression of which is restricted to late phases of the lytic viral reproductive cycle, which indicates a possible role in viral dissemination. vCCL4 binds to CCR2 in T cells, and causes $Ca^{2+}$ mobilization as efficiently as does its endogenous ligand, MCP 1/CCL2. vCCL4 also functions as a chemo-attractant for CCR2-expressing cells, which include macrophages and monocytes, conceivably to infect them and to establish latency.

Molluscum contagiosum virus (MCV), a poxvirus that produces benign cutaneous lesions, encodes a CC-chemokine homologue that is designated MCV chemokine homologue (MCCH) or MC 148. Like the KSHV-encoded vMIP-I, MC148 binds to the chemokine receptor CCR8. However, MC148 has a truncated N-terminus, which comprises a region that is required for proper binding and receptor activation, and so it functions as a CCR8 antagonist. MC148 specifically blocks monocyte infiltration and the function of dendritic cells, which might help to explain the prolonged absence of an inflammatory response in skin tumors that harbor replicating MCV.

*Virally encoded chemokine-binding proteins* - Hijacking the intracellular signaling pathways that are activated by GPCRs can facilitate viral pathogenesis. However, chemokine receptors are also essential for the host immune response and they can protect cells from viral infection. It is therefore not surprising that viruses have evolved proteins that specifically 'turn off' these GPCRs. This strategy is exemplified by the virally encoded chemokine-binding proteins (CKBPs, as exemplified in Fig. 1). These show no sequence similarity to any known host proteins, and yet bind with high affinity to cellular chemokines and inhibit their interaction with cognate receptors. The observation that viruses produce and deploy proteins that modulate or inhibit the normal function of chemokine receptors underscores the significance of their signaling pathways to the host immune response during viral infection. As discussed above, poxviruses encode a repertoire of proteins that are involved in immune evasion and immune modulation, including CKBPs. The myxoma virus encodes two such proteins, T1 and T7. T1 binds with high affinity to many CC chemokines, but with low affinity to CXC chemokines. By sequestering cellular chemokines T1 has been shown to block human monocyte migration. T7 is a more promiscuous CKBP that can bind to, and inhibit the activity of members of CC and CXC classes of chemokine, and also seems to function by preventing the formation of an external chemokine gradient, which inhibits monocyte chemotaxis. Open reading frames for GPCRs, virally encoded cytokines, and CKBPs that are found in human herpesviruses are also highly conserved in animal herpesviruses, including those that infect mice, rats, horses, and primates. This supports their biological relevance and has provided useful experimental models to help elucidate the function of these molecules *in vivo*. For example, the murine herpesvirus MHV-68, which is highly related to human α-herpesviruses HHV8 and ebola virus (EBV), encodes an abundantly secreted protein, M3, which binds to chemokines of several classes, including the CC, CXC, C, and CX3C chemokines, and which prevents them from signaling through GPCRs. Mutants of MHV-68 that lack M3 showed that the amplification of latently infected cells (cells that survive and proliferate and produce few viral progeny), which normally drives MHV-68-induced infectious mononucleosis, failed to occur. So, in the absence of M3, MHV-68 was unable to establish a normal latent viral load and was less pathogenic. This raises the prospect that HHV-8 and EBV may also encode CKBPs that might similarly function to sequester cellular chemokines and thus promote viral pathogenesis.

Example 1

**Direct detection of viral infection using a RWG biosensor; Signaling events involved in adenoviral entry** Signal transduction is emerging as an important regulator of early virus-host interactions. As mentioned herein, two mechanisms account for virus-induced cell signaling: 1) the activation of surface receptor; and 2) the accumulation of viral-encoded cellular signaling molecules in a target cell (including receptors). Activation of a viral receptor can, for example, stimulate or counteract cellular antiviral defenses, enhance apoptosis, or facilitate virus entry and production. Furthermore, viruses have been shown to stimulate the host inflammatory response, resulting in production of pro-inflammatory cytokines and chemokines, and activation of a number of signal transduction pathways.

Adenovirus vectors are known to result in the activation of the host inflammatory response and modulation of signal transduction pathways, including activation of MAP kinases and phosphatidylinositol 3-kinase (PI3-kinase). Adenovirus has been shown to stimulate the host inflammatory response, resulting in the production of pro-inflammatory cytokines and chemokines, and the activation of a number of signal transduction pathways including MAP kinases, focal adhesion kinase, and P13-kinase. Fig. 2 shows a schematic of exemplary signal events in adenoviral cell entry. Adenovirus-integrin interactions 210 induce FAK phosphorylation/activation 220. However, this event does not seem to be particularly important for virus entry. Instead, activation of phosphatidylinositol-3-OH kinase 230 and Rho family GTPases 235 serves to promote adenovirus entry. Adenovirus entry into cells is initiated by binding of the virus to its cell surface receptors. First, adenovirus fibre protein knob domain (Figure 2) bound to cell surface receptors the coxsackie-adenovirus receptor (CAR) or MHC class I$\alpha$2 domain. In addition, interactions between the adenovirus pentons and cell-surface integrins such as $\alpha$v$\beta$3 and $\alpha$v$\beta$5 have also been shown to facilitate the infection and promote adenovirus internalization. Soon after the binding, adenoviruses are assembled into clathrin coated pits, and in endocytic vesicles termed endosomes. In less than 5 minutes of the initial binding of adenoviruses to the cell surface, adenoviruses can be observed in endosomes. Adenovirus escapes from endosomes into the cytosol, then traverse towards the nucleus using the microtubule system. The journey of adenovirus from cell surface to the nucleus is completed in about 30 minutes, indicating a rapid rate of adenoviral uptake.

These responses occur early after virus binding and are independent of viral gene transcription. Adenoviral particles activate host innate immune responses *in vivo* and *in vitro.* The activation of host inflammatory genes is mediated by the adenovirus capsid and is independent of viral gene transcription. Adenovirus vectors interact with and activate numerous different cell types, including leukocytes, endothelial, and epithelial cells. In non-hematopoietic cells, studies show that adenovirus vectors activate a transcription factor NF$\kappa$B and a number of signaling pathways such as extracellular signal-regulated kinase (ERK) and p38 during viral cell entry, which results in the up-regulation of immunoregulatory genes, including those for cytokines, chemokines, and adhesion molecules. CXCL10 is a chemokine that is rapidly up-regulated in models of adenovirus vector-induced inflammation. Recent studies have confirmed that CXCL10 plays a pivotal role in the recruitment of T cells into the liver after adenovirus infection. The induction of CXL10 thus serves as a useful marker of cellular activation and the host immune response to adenorirus vectors *in vitro* and *in vivo.* Adenoviral vector entry in non-hematopoietic cells first occurs through a high-affinity interaction between the adenovirus fiber knob region and the coxsackie virus-adenovirus receptor (CAR). The binding of the fiber knob to CAR is thought not to trigger signaling events but rather to disrupt the integrity of host cell junctions to facilitate virus internalization. Following initial binding, peptidic Arg-Gly-Asp (RGD) motifs in the adenovirus penton base protein bind to $\alpha$v integrins, which facilitates virus internalization. Adenovirus vector induced signal transduction and chemokine gene expression correlated with reduced cellular entry but still occurred in the absence of CAR and integrin binding. Activation of the host inflammatory mechanisms occurred in a post internalization step of adenovirus vector cell entry. Binding of capsid RGD motifs to av integrins not only facilitates virus internalization but also triggers several integrin-induced signaling pathways, including the phosphoinositide-3-OH kinase (PI3K) pathway. The PI3K-Akt pathway has been demonstrated to positively regulate NF$\kappa$B. In non-hematopoeitic cells, adenovirus vectors stimulate host inflammatory genes by activating at least two signal transduction pathways at different steps during virus-cell attachment and entry: the PI3K-Akt pathway, activated through capsid-dependent binding to cell surface integrins; and the mitogen-activated protein kinase pathway, activated post internalization.

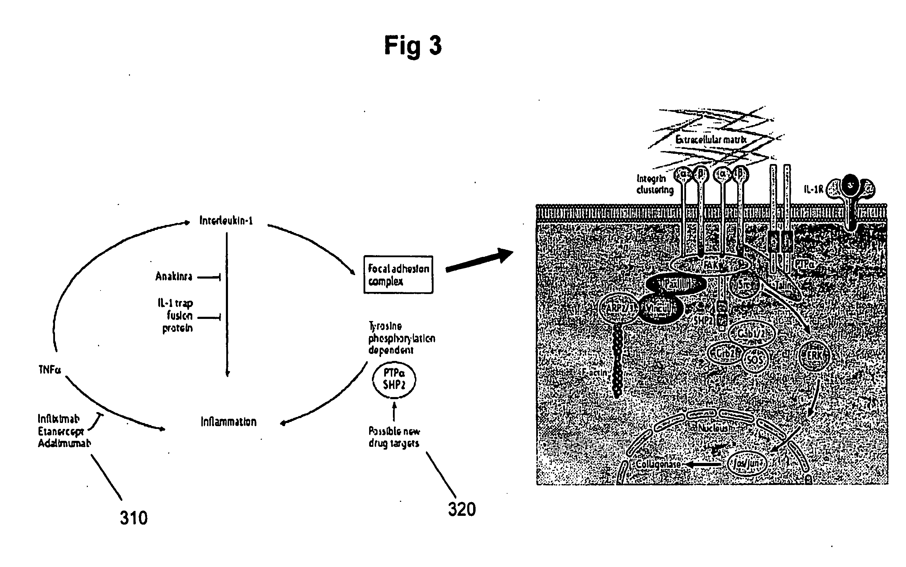

Fig. 3 shows a schematic of a *loci* for possible therapeutic intervention in the treatment of inflammation, for example, the site of action of existing 310 and novel 320 therapeutics. Inflammation is a basic response to a variety of external or internal insults, such as infectious agents, physical injury, hypoxia, or disease processes in nearly any organ or tissue in the body. Inflammation entails the four well-known symptoms redness, heat, tenderness/pain, and swelling that characterize so many common diseases and conditions. Small molecule therapeutics that target GPCRs involved in inflammatory processes have been developed, since GPCRs help modulate the inflammatory process. Thus, one can apply screening technologies of the disclosure to these targets to identify small molecules that could activate or inhibit these GPCRs. Some of the targeted GPCRs can be expressed on T- and B-cells and macrophages, and may be important in the modulation of key cytokines that mediate inflammatory processes such as tumor-necrosis factor alpha (TNF-alpha), an important pro-inflammatory mediator in diseases such as rheumatoid arthritis.

Chemokines are small proteins that regulate the immune system, particularly chemotaxis (cell migration due to a chemical gradient). To date, four families of chemokines have been identified, consisting of over 50 proteins that bind to one or more of the 13 known chemokine receptors. Recent studies have demonstrated a role for chemokines in the pathogenesis of several inflammation-associated diseases, including asthma and atherosclerosis. A family of peptides and small molecules that exhibit the ability to inhibit migration of inflammatory cell has been identified. While the majority of reported chemokine inhibitors are specific for one or a selected group of chemokines, these new compounds exhibit broad chemokine inhibitory activity and have demonstrated efficacy in a variety of animal models, including those for atherosclerosis, asthma, stroke, endotoxemia, and dermal inflammation.

Numerous recent investigations have pointed to a key role of the pro-inflammatory, pleotropic cytokines tumor-necrosis factor-α (TNFα), IL-6, and IL-1, in host defense and inflammatory disease processes. TNF and IL-1 over-expression has been found in disease target tissue and in the blood of patients with acute and chronic inflammatory diseases. It has been suggested that TNF-alpha and IL-1 are crucial in these diseases. Over the last 10 years several approaches to inhibit TNF-alpha and, in one case, IL-1 activity, have been developed by the biotechnology and pharmaceutical industries. Several approaches have been developed for the pharmacological regulation of IL-1 and TNFα signals by either receptor blockade, interference with cytokine function, or inhibition of the production, processing and release of the cytokine. Drugs that block the pro-inflammatory cytokines TNF-α and IL-1 can improve outcomes for rheumatoid arthritis and other inflammatory diseases but many patients remain refractory to treatment. The exploration of the crucial molecules required for receptor clustering, and therefore signal transduction, offers new targets and scope for anti-inflammatory drug development. Focal adhesions are increasingly recognized for their role in signal transduction (Fig. 3). Indeed, these complex structures are now known to form multi-meric signaling complexes that orchestrate essential aspects of cell behavior including cell shape, motility, proliferation, apoptosis, and responses to environmental cues such as physical forces, growth factors, and inflammatory stimuli. In addition to integrins and structural cytoskeletal proteins such as vinculin, talin, tensin, paxillin, zyxin, and αactinin, focal adhesions contain a diverse array of signaling molecules (i.e., >50), including protein kinases and phosphatases, small GTPases and associated regulatory molecules, and adaptor molecules that mediate key protein-protein interactions. Some of these focal adhesion molecules are known to be directly involved in IL-1 signaling because engagement of IL-1R by IL-1β leads to phosphorylation of the scaffold protein talin and focal adhesion kinase. The repertoire of focal adhesion proteins that are involved in IL-1 signaling is dependent on the extent of focal adhesion maturation following initial cell attachment. A more global view of the large number of potential signaling regulatory molecules in focal adhesions indicates broad scope for pharmacological target discovery both in cancer and, most notably, inflammation. Focal adhesions often mature through a series of stages (focal contacts, focal adhesions and fibrillar adhesions), each with a distinctive appearance and molecular composition. In the absence of exogenous stimuli, focal adhesions develop and mature slowly over many hours. Exogenous stimuli can profoundly modulate this process; exposure to growth factors such as platelet-derived growth factor (PDGF), cytokines such as IL-1β, and mechanical forces can promote maturation and dynamic remodeling of focal adhesions. Many of these responses are regulated by tyrosine phosphorylation-dependent events that are crucial to the formation, maturation, and dynamic remodeling of focal adhesions as well as modulation of downstream signaling pathways. In the context of drug discovery for blockade of IL-1 signals, prevention of focal adhesion maturation with peptides that disperse focal adhesions can block IL-1 signaling which can lead to extracellular signal-regulated kinase (ERK) activation. These data illustrate the potential for using cell adhesions and cell adhesion-related proteins as targets for novel therapeutics. Focal adhesions contain numerous tyrosine phosphorylated proteins including paxillin, focal adhesion kinase, and Src family kinases. The latter have pivotal and multifaceted roles in focal adhesion formation and maturation (Fig. 3). In the context of IL-1 signaling, tyrosine phosphorylation of FAK in response to IL-1 is required for signal transduction in fibroblasts, underscoring the importance of tyrosine phosphorylation in regulation of IL-1 signal transduction. Indeed, tyrosine phosphorylation is pivotal in the formation, maturation, and dynamic remodeling of focal adhesions as well as in the modulation of many downstream signaling pathways including IL-1. As protein tyrosine phosphatases (PTPs) are known to modulate pivotal signaling pathways involved in immune, inflammatory, and fibrotic responses, selective modulation of these pathways by strategies that target PTPs is desirable. There are precedents for targeting tyrosine kinases with molecular therapeutics. Recent studies have demonstrated the effectiveness of tyrosine kinase inhibitors in the treatment of a variety of cancers. For example, imatinib mesylate (Gleevec; Novartis), an oral tyrosine kinase inhibitor that targets BCR-Abl, c-Kit, and PDGF receptors a and β (both tyrosine kinases), has been shown to be effective in the treatment of chronic myelogenous leukemia and a variety of other cancers. Strategies that target the EGF receptor with small molecule tyrosine kinase inhibitors such as gefitinib (Iressa; AstraZeneca) and EKB-569 (Wyeth-Ayerst), or which use blocking monoclonal antibodies such as matuzumab (EMD Pharmaceuticals), have also shown promise in the treatment of several cancers. The small molecule tyrosine kinase inhibitor SU5416 (semaxanib; Sugen), which targets the vascular endothelial growth factor receptor, has also shown promise as an anti-neoplastic treatment. Additionally, agents that target tyrosine kinases, such as JAK family members, are being developed as immuno-modulatory agents for the treatment of immune and inflammatory disorders. Compared with the therapeutic use of tyrosine kinase inhibitors, much less is known about therapeutic approaches that target PTPs. Nonetheless, the broad-range PTP inhibitor vanadate and its derivatives have shown promise in the treatment of diabetes mellitus in both animal models and humans. Selective modulation of signaling pathways triggered by IL-1, especially those that are focal adhesion-dependent, is another therapeutic strategy for the amelioration of inflammatory tissue injury. As this represents relatively uncharted territory, researchers would do well to draw on the experience obtained with developing PTP inhibitors for other diseases, such as diabetes, to expedite the development of PTP inhibitors for the treatment of inflammatory disorders. In this regard, a small-molecule inhibitor of SHP2, NCS-87877, has recently been described that binds to the catalytic cleft of SHP2, thereby inhibiting its phosphatase activity. This compound selectively inhibited EGF-induced ERK activation in cultured cells without affecting ERK activation by other stimuli, raising the possibility of its use in modulating SHP2-dependent signaling pathways that mediate inflammatory tissue injury. Clearly, such an approach must be undertaken judiciously because PTPs such as SHP2 and PTPa participate in signaling pathways that regulate physiologically important processes in structural and immune cells. However, the local delivery of therapeutic agents for brief periods of time into an inflammatory milieu such as the joint or the lung might allow some selectivity in the modulation of signaling pathways.

## Materials and Methods

*Reagents* - Latrunculin A and cytochalasin B were purchased from Sigma Chemical Co. (St. Louis, MO). Cell permeable dynamin inhibitor peptide control (DIPC) was obtained from Tocris Chemical Co. (St. Louis, MO). Adenoviral particles (Ad-CMV-eGFP) were purchased from Vector Biolabs (Philadelphia, PA). Cell-culture-ready Corning® Epic™ 96well biosensor microplates were obtained from Coming Inc (Corning, NY). HeLa cell line was obtained from Americal Type Cell Culture.

*Cell Culture* - HeLa cells were grown in Earle minimum Essential medium (EMEM) supplemented with 10% fetal bovine serum (FBS), 4.5g/liter glucose, 2 mM glutamine, and antibiotics. ~$10^4$ HeLa cells suspended in 200 $\mu$l the appropriate medium containing 10% FBS were placed in each well of a 96well biosensor microplate, and were cultured at 37°C under air/5% $CO_2$ until about 95% confluency was reached.

*Optical biosensor measurements* - Corning® Epic® angular interrogation system with transverse magnetic or p-polarized $TM_0$ mode (as described in, for example, U.S. Patent Publication No. US-2004-0263841, U.S. Patent Publication No. US-2005-0099622, and U.S. Patent Publication No. US-2005-0236554.) was used. After culturing the cells were washed twice and maintained with 100 microliters 1x HBSS (1x regular Hank's balanced salt solution, 20 mM HEPES buffer, pH 7.0). Afterwards, the sensor microplate containing cells was placed into the optical system, and the cell responses were recorded before and after addition of a solution. For compound studies, the cells in each well were pretreated with a compound solution of 50$\mu$l or the 1x HBSS until a steady phase (i.e., no obvious mass redistribution) was reached (generally within one hour), before viral particle-containing solution of 50 microliters was introduced. All studies were carried out at room temperature with the lid of the microplate on except for a short period of time (about seconds) when the solution was introduced, in order to minimize the effect of temperature fluctuation and evaporative cooling.

## Results and Discussions

Adenoviral receptors are expressed on most cell types including epithelial, neuronal, fibroblast and muscle cells. The only known cell types deficient in adenoviral receptors are primary hematopoietic cells, including CD34+ stem cells. However, adenoviruses can stay in the episomal state in some lymphoid cells. While the mechanism of this latency is not clear, it raises the possibility of an alternate receptor or perhaps other means of adenovirus entry into cell types.

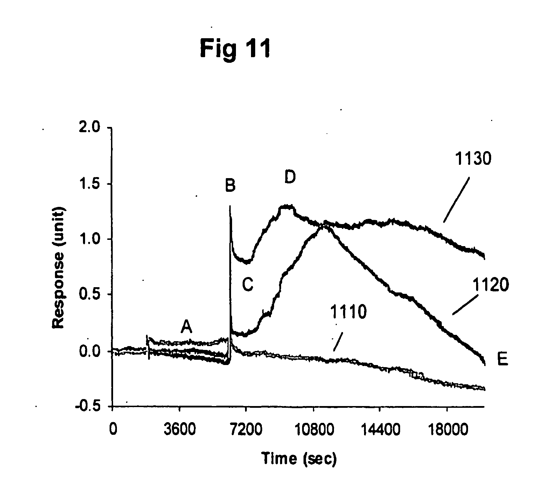

HeLa cells are known to be able to be infected by adenoviruses, and chosen as a model to study the viral entry and signaling of adenovirus. Using a Corning® Epic® angular interrogation system, adenoviruses can be directly detected using cell-based assays. Fig.11 shows the real time kinetics of the cell responses induced by two different concentrations of virus (multiplicity of infection of 3,000 (MOI 3000) (1130), and 12,000 MOI (1120)), in comparison with that induced by the buffer only (1110). As expected, the buffer (1xHBSS) resulted in a little downward drifting signal. On the other hand, HeLa cells respond to adenovirus in a dose-dependent manner. When the concentration of adenovirus is at 12,000 MOI, the resultant DMR consists of three major phases: a N-DMR event with a deceasing signal lasting about 30 min (point B to C), a subsequent P-DMR with an increasing signal lasting about 45 min (point C to D), and a N-DMR event lasting several hours (point D to E). There is an initial rapid P-DMR signal (point A to B), right after the addition of virus solution, which is due to the difference in refractive index between the cell medium and the virus solution (higher refractive index). Such signal is referred to bulk index signal which only lasts for a very short period of time (<1 min).

At lower doses (e.g., MOI 3000), the virus triggered a DMR signal, which overall dynamics is similar to that induced by a dose of virus equivalent to MOI 12000 virus, but with significantly different kinetic characteristics. Compared to the DMR signal induced by MOI 12000, the overall dynamics of the DMR signal induced by lower concentrations of virus also exhibits three major phases: a N-DMR signal with much smaller amplitude, a subsequent P-DMR signal with much slower kinetics, and a N-DMR signal with much slower kinetics. In addition, the transition from the P-DMR phase (C-D) to the N-DMR phase (D to E) is much delayed. These results suggested that adenovirus mediated significant DMR signal; such DMR signal can be used as an indication of viral infection.

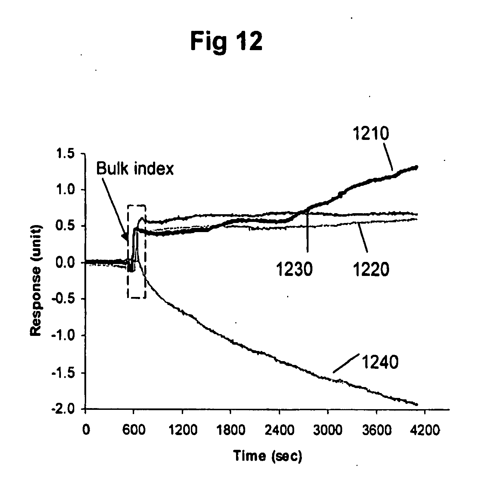

Upon binding to cell surface receptors such as coxsackie adenovirus receptors, adenoviruses undergo internalization and trafficking, as well as mediate cell signaling. It is known that cellular microtubules and actin filaments play important roles in virus trafficking. Drugs such as vinblastin and cytochalasin B, which disassemble these filaments, have been shown to block the trafficking of adenovirus in cells. Thus, several modulators have been chosen to pretreat HeLa cells; their ability to modulate the virus-induced DMR signal is examined. Results are summarized in Fig. 12. The modulators include: DIPC (dynamin inhibitory peptide control), and two actin filament-disrupting toxins latrunculin A and cytochalasin B. The DIPC can block the activity of dynamin, a critical intracellular protein that plays important roles in viral-receptor complex endocytosis. Compared to the positive control 1210 (in which the HeLa cells were pretreated with 1xHBSS buffer only, followed by the addition of MOI 6000 adenovirus), the pretreatment of cells with actin disruption agents as cytochalasin B 1230 and latrunculin A 1220 (Fig. 12) clearly inhibited the P-DMR signal within the assay time (about 1 hour). Since the actin filaments play important roles in receptor trafficking, these results suggest that the P-DMR event is downstream to the viral entry. On the other hand, DIPC, an inhibitor of the GTPase dynamin that competitively blocks binding of dynamin to amphiphysin, completely blocks the P-DMR event (Fig. 12, curve 1240). This suggests that the endocytosis of virus is important to the P-DMR signal.

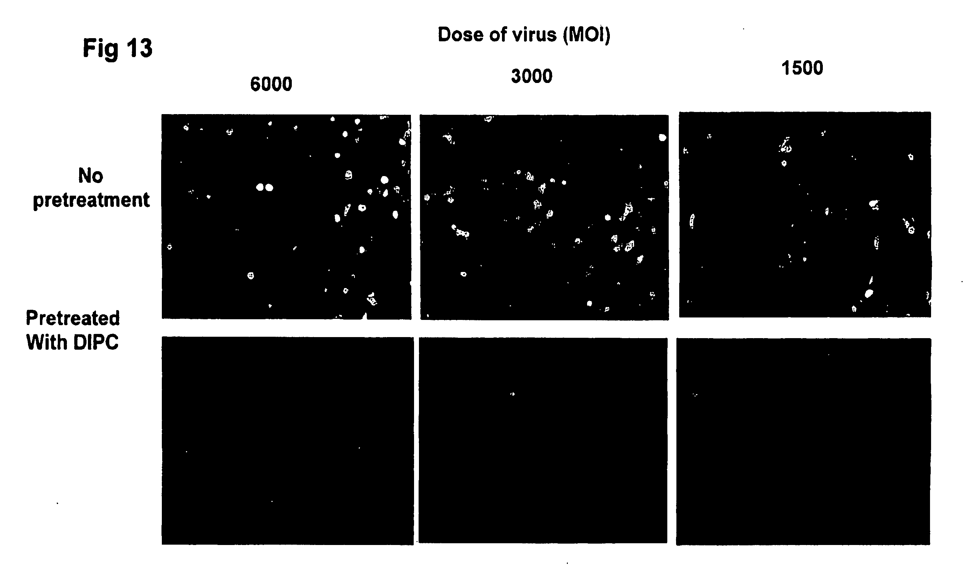

After the biosensor assays with Epic angular interrogation system, the microplate containing cells was incubated at standard cell culturing condition for overnight. Afterwards, the expression of green fluorescence proteins (GFP) was examined using fluorescence microscopy. This serves to confirm the modulation profiles of these inhibitors on viral infection, since the adenoviral vectors used in this assay, contain GFP gene. Results showed that 20h post infection, HeLa cells only infected with adenovirus-GFP showed significant fluorescence due to the expression of GFP. In contrast, a pretreatment of Hela cells with DIPC did completely prevent the viral infection, as shown by the lack of GFP expression in infected cells (Fig.13).

In the direct approach it has been demonstrated that using RWG biosensor, it was possible to directly monitor the viral infection in host cell lines. Fig. 4A illustrates the direct detection method, which is based on pathogen-induced DMR signal. The direct detection method can be configured, for example, to include an optical biosensor 400, such as a wave-guide made of, for example, glass, an interrogator 410, including for example, a broadband light source or beam and a receiver for receiving the interrogated beam, having a detection volume 415, cell surface adhesion sites 417, members, or like means, an adhered cell 420, having a DMR cell component 422, a cell nucleus 425, and an optional extracellular pathogen 430 such as a viral particle. The direct method is advantaged by having a direct, rapid response time but is disadvantaged by having relatively low sensitivity, for example, having a threshold of about the 1,000 viral particles or more. The direct method can be accomplished using, for example, a Corning® Epic® system.

## Example 2

**Mapping of the impact of viral infection on cell signaling networks of living cells using panel of modulators** To further improve the sensitivity of biosensor cell-based assays a second indirect approach was investigated which explored a virus's propensity to hijack the cell signaling. The indirect approach used a panel of "markers", each of which modulates at least one distinct cellular target, such as a receptor, and thus subsequently triggers different cell signaling pathway(s) (e.g., $Ca^{2+}$ pathway, MAPK pathway, adhesion pathway, cAMP pathway, and like pathways). The impact of pathogen intrusion, such as viral infection, on the marker-induced DMR signals can be used as a reliable measure.

Fig. 4B illustrates the indirect detection method which is based on pathogen-induced changes in cell signaling. The indirect detection method can be configured similarly to the direct method as illustrated in Fig. 4A, including for example the optical biosensor 400, the interrogator 410 having the detection volume 415, cell surface adhesion sites 417, an adhered cell 420 having a DMR cell component 422, a cell nucleus 425. In the indirect detection method intracellular structures 432, nuclear structures 427, or both, may undergo changes as a result of pathogen intrusion upon the cell's signaling pathway(s), for example, as a result of pathogen interaction with cell surface structures, such as a GPCR 440, RTK (receptor tyrosine kinase) 445, and like structures, or for example where the pathogen 431, or a component or artifact thereof, has entered the cell's nucleus 425. The indirect method is advantaged by being a highly sensitive and rapid assay having a detection threshold of from about 1 to about 100 viral particles but is disadvantaged by having a longer incubation or infection period compared to the direct method. The indirect method can be accomplished using, for example, a Corning® Epic® system.

In one example the adenoviral infection on A431 cells was measured. A series of markers were examined including: EGF (targeting EGFR and MAPK pathway and cell adhesion pathway), Bradykinin (targeting Gq and $G_s$ pathway), SFFLR-amide (targeting Gq and $G_{12/13}$ pathway), SLIGKV-amide (targeting Gq and $G_{12/13}$ pathway), forskolin (targeting cAMP pathway), A23187 (targeting $Ca^{2+}$ pathway) and Epinephrine (targeting $G_5$ pathway and cell adhesion pathway). EGF is a natural ligand of epidermal growth factor receptor. Bradykinin is a natural agonist of bradykinin B2 receptor. SLFFLR-amide is an agonist of protease activated receptor subtype 1 (PAR1), and SLIGKV-amide an agonist of protease activated receptor subtype 2 (PAR2). Epinephrine is a natural agonist of beta2-adrenergic receptor. All these receptors are endogenously expressed in A431 cells. In addition, forskolin is an activator of adenylate cyclases, a class of cellular enzyme to convert ATP into cAMP. A23187 is a $Ca^{2+}$ ionophore, which results in a $Ca^{2+}$-dependent cell apoptosis. The effect of adenoviral infection on activation of these pathways was measured. Evidence of an effect on cell signaling induced by the adenovirus was also evaluated on an Epic® system.

**Materials and Methods**

*Reagents* - SFLLR-amide, bradykinin, and SLIGKV-amide were purchased from Bachem (King of Prussia, PA). Epinephrine, A23187, forskolin and EGF were obtained from Sigma Chemical Co. (St. Louis, MO). Corning® Epic™ 384well biosensor microplates were obtained from Coming Inc (Coming, NY). In the sensor microplate, each well contains a RWG sensor consisting of a thin film of dielectric material on the grating presenting substrate.

*Cell Culture* - Human epidermoid carcinoma A431 cells were obtained from American Type Cell Culture. For A431 cell culturing, A431 cells were grown in Dulbecco's modified Eagle's medium (DMEM) supplemented with 10% fetal bovine serum (FBS), 4.5g/liter glucose, 2 mM glutamine, and antibiotics. about $1-2x10^4$ cells at passage 3 to 5 suspended in 50$\mu$l the growth medium were placed in each well. After cell seeding, the cells were cultured at 37°C under air/5% $CO_2$ until about 95% confluency was reached (about 2 days). Afterwards, the serum medium was replaced with the serum-free DMEM medium, and at the same time adenovirus particles were introduced. The resultant cells were subject to continuous culturing for overnight.

*MRCAT (Mass Redistribution Cell Assay Technologies) assays* - Instead of the angular interrogation system previously used, the Corning® Epic® Label-free, wavelength interrogation system with transverse magnetic or p-polarized $TM_0$ mode was used in this study. For DMR assays, the cells in each well were maintained with the DMEM medium of 40$\mu$l, and were pretreated with a compound solution of 10$\mu$l or 1x HBSS until a steady phase (i.e., no obvious mass redistribution) was reached (generally within one hour), before the activator solution of 10$\mu$l was introduced. All studies were carried out at room temperature with the lid of the microplate on except for a short period of time (~seconds) when the solution was introduced, in order to minimize the effect of temperature fluctuation and evaporative cooling.

**Mass Redistribution Cell Assay Technology (MRCAT)**

In commonly-owned, copending PCT application, entitled "Label-Free Biosensors and Cells," Y. Fang et al., PCT App. No. PCT/US2006/013539 (Pub. No. WO 2006/108183), published Dec. 10, 2006, there is disclosed a non-invasive and manipulation-free cell assay methodology referred to as Mass Redistribution Cell Assay Technology (MRCAT). MRCAT uses an optical biosensor, particularly resonant waveguide grating (RWG) biosensor, to monitor the ligand-induced dynamic mass redistribution within the bottom-most portion of adherent cells. The DMR signal obtained represents an integrated cellular response, which resulted from a ligand-induced dynamic, directed, and directional redistribution of cellular targets or molecular assemblies. MRCAT permits the study of cell activities, such as signaling and its network interactions, and can also enable high throughput screening of ligand candidate compounds against endogenous receptors or over-expressed receptors in engineered cells or cell lines.

Since the optical biosensor exploits a typical short evanescent wave to probe the cellular activities and signaling, the cells are generally required to bring to contact with the surface of a biosensor. This can be achieved by several mechanisms. For adherent cells, cells can be directly cultured onto the surface of a biosensor. For weakly adherent cells, cells can be directly cultured onto the surface of a biosensor whose surface consists of a material supporting the anchorage of the cells (e.g., extracellular matrix materials such as fibronectin, lamin, collagen, gelatin; or polymeric materials such as polylysine and amine-reactive polymers). For suspension cells, the cells can be brought into contact with the surface of a biosensor whose surface consists of reactive moieties (such as amine-reactive polymer to interact with the cell surface proteins and thus couple the cells with the surface, or antibodies to interact specifically with the cell surface proteins and thus anchor the cells onto the sensor surface).

MRCAT starts with the interaction or contact of cells with the surface of a biosensor. Typically, cells are cultured directly onto the surface of a RWG biosensor. Exogenous signals can mediate the activation of specific cell signaling, in many instances resulting in dynamic redistribution of cellular contents equivalent to dynamic mass redistribution (DMR). If signaling occurs within the sensing volume (i.e., the penetration depth of the evanescent wave) then the DMR can be manifested and monitored in real time by a RWG biosensor. Because of its ability for multi-parameter measurements, the biosensor has potential to provide high information content for cell sensing. These parameters include the angular shift (the most common output), the intensity, the peak-width-at-half-maximum (PWHM), the area, and the shape of the resonant peaks. The position-sensitive responses across an entire sensor can provide additional useful information regarding to the uniformity of cell states, for example, density and adhesion degree, as well as the homogeneity of cell responses for cells located at distinct locations across the entire sensor.

The DMR signals can yield valuable information regarding novel physiological responses of living cells. Because of the exponential decay of the evanescence wave tail penetrating into the cell layer, a target or complex of a certain mass contributes more to the overall response when the target or complex is closer to the sensor surface as compared to when it is further from the sensor surface. Furthermore, the relocation of a target or complex towards the sensor surface results in an increase in signal, whereas the relocation of a target or complex that moves away from the sensor surface leads to a decrease in signal. The DMR signals mediated through a particular target were found to depend on the cell status, such as degree of adhesion, and cell states, such as proliferating and quiescent states.

Because of the short sensing volume of commonly available optical biosensors such as RWG and SPR, the biosensor-based cell assays depend on close proximity of cells with the sensor surface. In addition, attachment of cells, growth of cells, or both, can be significant factors in the success of the present cell-based biosensor and its assay methods. The modified biosensor surfaces of the disclosure should be biocompatible with and support the attachment and growth of a wide variety of cell lines. Cells adhered to the biosensor surface can withstand manipulations such as washing and reagent dispensing.

**Viral Hijacking of cell signaling monitored on Epic® system using MRACT**

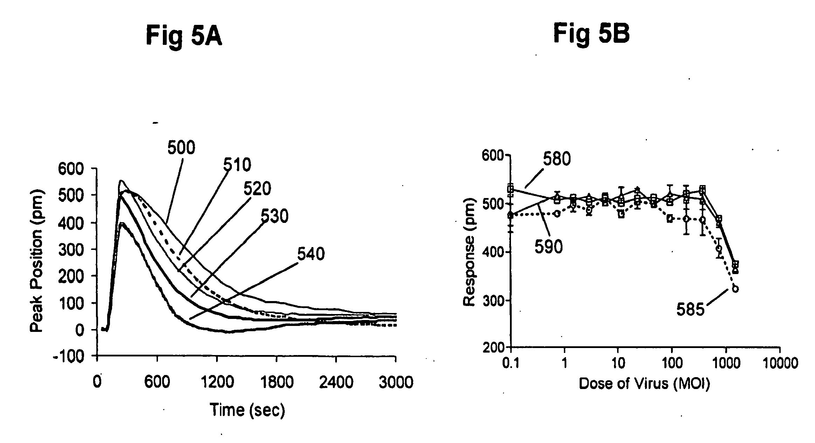

A431 cells were cultured onto the unmodified surface of RWG biosensor and were then infected overnight in serum free medium with various doses of Adenovirus-GFP. The doses of virus (multiplicity of infection (MOI)) used in this experiment were comprised of from 0 to about MOI 6,000. At 20 hrs post infection (PI) the A431 cells were washed with HBSS and real time kinetics of the cell responses were recorded before and after stimulation with 32 nM EGF (targeting EGFR and MAPK pathway and cell adhesion pathway), 25 nM epinephrine (targeting $G_s$ pathway and cell adhesion pathway), 32 nM of bradykinin (targeting $G_q$ and $G_s$ pathway), 25 microM of SFFLR-amide or 25 microM of SLIGKV-amide (both targeting Gq and $G_{12/13}$ pathways) using the Epic® system and illustrated in Figs 5 to 7. At MOI 3,000 and 6,000, the A431 cells detached from the surface and washed out before the assay. Therefore no significant data were collected at these viral concentrations. The amplitude of N-DMR signals (for EGFR) or the P-DMR signals (for bradykinin, SFFLR-amide, SLIGKV-amide and epinephrine) was plotted as function of the dose of adenoviral particles used in the assay.

Figs. 5A and 5B, respectively, show exemplary biosensor measurements and results of an adenoviral infection mediated Gq signaling interference. Fig. 5A shows the measurements of the wavelength of resonant peak as a function of time for various doses of adenovirus and their impact on Gq signaling: Mock (the control, meaning no viral infection) 500, AdenoGFP MOI 192 (510) (meaning the cells are pretreated and infected with the virus at MOI 192), AdenoGFP MOI 384 (520), AdenoGFP MOI 768, (530), and AdenoGFP MOI 1536 (540). Fig. 5B shows biosensor P-DMR amplitudes as a function of virus dose when bradykinin 580, SLIGKV-amide 585, or SFFLR-amide 590 were present.

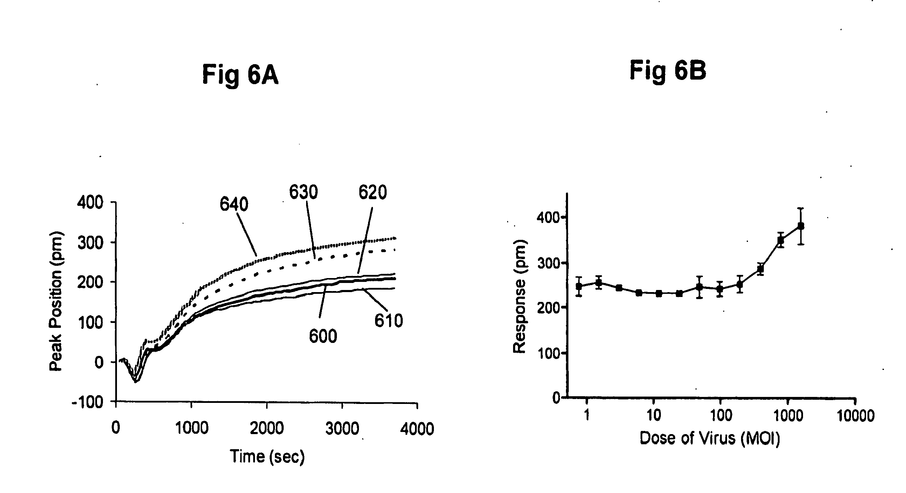

Figs. 6A and 6B, respectively, show exemplary biosensor measurements and results of an adenoviral infection mediated $G_s$ signaling interference. Fig. 6A shows biosensor peak position measurements as a function of time for various doses of adenovirus and their impact on $G_s$ signaling: Mock (600), AdenoGFP MOI 192 (610), AdenoGFP MOI 384 (620), AdenoGFP MOI 768 (630), and AdenoGFP MOI 1536 (640). Fig. 6B shows biosensor P-DMR amplitudes of the epinephrine-induced DMR signal in A431 cells as a function of virus dose.

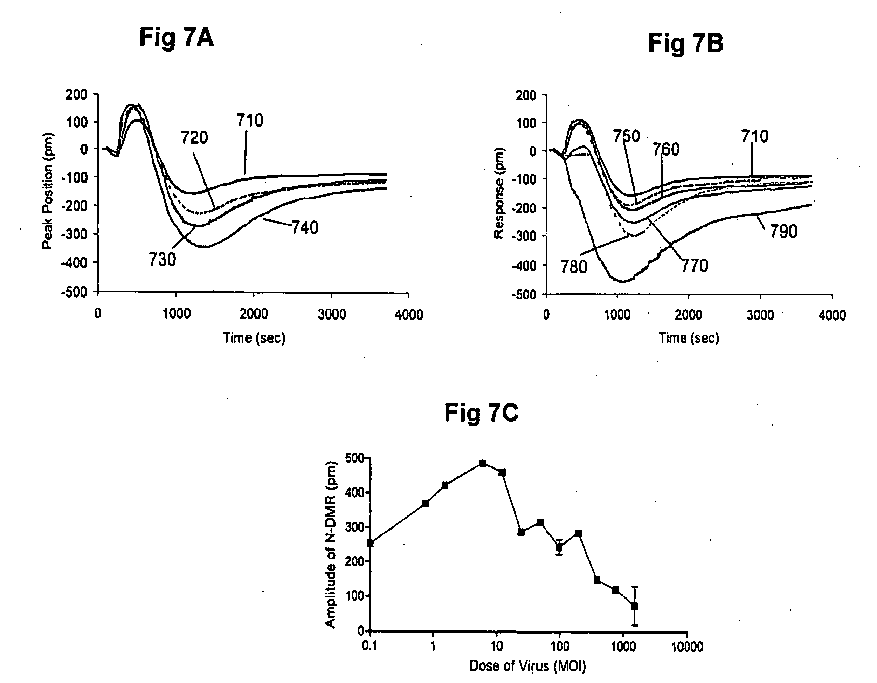

Fig. 7A and 7B show the effect of adenoviral infection on the DMR signal of A431 cells induced by EGF 32 nM. A431 cells were infected with different concentrations of adenoviral particles, MOI of from about 1 to about 10 (Fig. 7A), and MOI of from about 20 to about 1,500 (Fig. 7B). Fig. 7A shows biosensor peak position measurements as a function of time for Mock 710, AdenoGFP MOI 0.75 (720), AdenoGFP MOI 1.5 (730), AdenoGFP MOI 6 (740). Fig. 7B shows biosensor peak position measurements as a function of time for mock control (710), AdenoGFP MOI 24, (750), AdenoGFP MOI 48 (760), AdenoGFP MOI 96 (770), AdenoGFP MOI 192 (780), AdenoGFP MOI 768 (790). Fig. 7C shows biosensor N-DMR amplitudes as a function of virus dose over the range of 0.1 to beyond MOI 1,000.

**$G_q$-coupled receptors** - Unique to $G_q$-coupled receptor signaling is the dramatic translocation of its signaling components, including several protein kinase C isoforms, GPCR kinase, β-arrestin, PIP-binding proteins, and diacylglycerol-binding proteins. Following receptor biology, our numerical analysis suggested that the protein translocation and receptor internalization are two primary resources for the DMR signatures observed for $G_q$-coupled receptor signaling. As shown in Fig. 5, adenoviral infection partially desensitized the Gq pathway at high doses (e.g., MOI 1,000) as shown by the significant decrease of P-DMR amplitude. The Gq pathway at doses below about 750 viruses/cell was not influenced by the adenoviral infection. Although SFLLR-amide and SLIGKV-amide also result in the signaling through G12/13, beside Gq. The $G_q$-mediated signaling dominates in both agonist-induced DMR signals. The similarity of adenoviral infection on the attenuation of the DMR signals mediated by the three agonists examined also indicates that the adenoviral infection at high doses primarily desensitizes the Gq signaling.

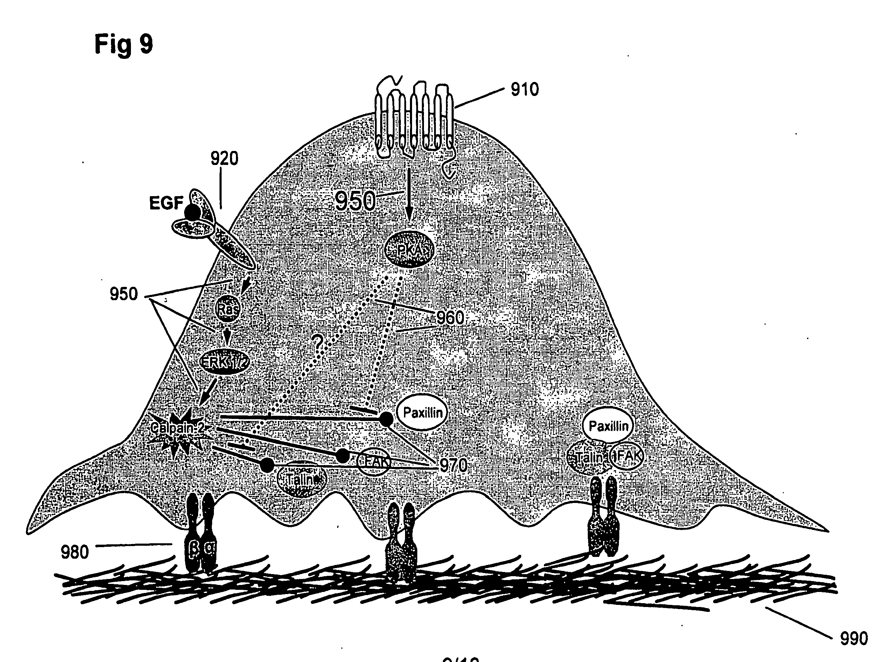

**$G_s$-coupled receptors** - β_2-adrenergic receptor (β_2AR) is a prototypical $G_s$-coupled receptor. Central to the β_2AR signaling is sequential activation of the receptor, G protein, and adenylyl cyclase at the plasma membrane, increased accumulation of a diffusible second messenger cAMP, and activation of PKA. Cell stimulation with epinephrine, an agonist of this receptor, results in a dose-dependent DMR signal in A431 - a cell line that presents large numbers of β_2AR, but not β_1AR. The DMR is characterized by a small N-DMR, followed by a significant P-DMR event (Fig. 6, mock control 600). Chemical-biology studies link the epinephrine-induced DMR to the cAMP/PKA pathway. Since the majority of downstream signaling components directly involved in the β_2AR signaling complexes, with the exception (thus far) of A-kinase anchoring proteins (AKAPs) and β-arrestins, are already compartmentalized at or near the cell membrane, the recruitment of intracellular targets to the activated receptors is much less pronounced than for EGFR or $G_q$-coupled receptor signaling. However, together with the rapid segregation of receptor signaling complexes into the clathrin-coated pits, the conversion of local ATP to cAMP and its subsequent diffusion away from the cell membrane compartments leads to a rapid and significant decrease in local mass. The convergence of these events leads to the initial N-DMR event. It is known that the PKA activation results in suppression of several kinases (e.g., FAK) involved in the cell adhesion complexes, and can lead to increased cell adhesion (Fig. 9). The increase in adhesion is the major contributor to the P-DMR event. In cells infected at doses higher than about MOI 350, a significant increase of the P-DMR induced by epinephrine was observed (Fig. 6, Compare mock control vs Adeno). In this instance adenoviral infection did have a positive effect on the Gs and cell adhesion pathway.

**EGFR signaling** - Epidermal growth factor receptor (EGFR) belongs to the family of receptor tyrosine kinases. Upon EGF stimulation, many events lead to mass redistribution in A431 cells — a cell line endogenously over-expressing EGFRs. A unique optical signature of this dynamic mass redistribution was identified and is described. EGF binds to and stimulates the intrinsic protein-tyrosine kinase activity of EGFR, which initiates a signal transduction cascade, principally involving the MAPK, Akt and JNK pathways. In quiescent cells obtained through 20 hr culturing in 0.1% fetal bovine serum, EGF stimulation lead to a DMR signal with three distinct and sequential phases: (i) a positive phase with increased signal (P-DMR); (ii) a transition phase, and (iii) a decay phase (N-DMR) (Fig. 7, mock control 710). Biochemical and cell-biology studies showed that the EGF-induced DMR signal is primarily linked to the Ras/MAPK pathway, which proceeds through MEK and leads to cell detachment. Two findings suggest that the P-DMR is mainly due to the recruitment of intracellular targets to the activated receptors at the cell surface. First, blockage of either dynamin or clathrin activity has little effect on the amplitude of the P-DMR event. Dynamin and clathrin, two downstream components of EGFR activation, play crucial roles in executing EGFR internalization and signaling. Second, the blockage of MEK activity partially attenuates the P-DMR event. MEK is an important component in the MAPK pathway, which first translocates from the cytoplasm to the cell membrane, followed by internalization with the receptors, after EGF stimulation. The N-DMR event however, may be due to cell detachment and receptor internalization. Fluorescent images show that EGF stimulation leads to a significant number of internalized receptors and cell detachment. It is known that blockage of either receptor internalization or MEK activity prevents cell detachment, and receptor internalization requires both dynamin and clathrin. This suggests that blockage of either dynamin or clathrin activity should inhibit both receptor internalization and cell detachment, while blockage of MEK activity should only inhibit cell detachment, but not receptor internalization. As expected, either dynamin or clathrin inhibitors completely inhibit the EGF-induced N-DMR (about 100%), while MEK inhibitors only partially attenuate the N-DMR (about 80%). Fluorescent images also confirm that blocking the activity of dynamin, but not MEK, impairs the receptor internalization.

We examined the effect of adenoviral infection on the EGFR, MAPK pathway and cell adhesion pathway, by measuring the effect of adenoviral infection on the response of A431 cells induced by EGF. As shown in Fig. 7, these specific pathways were affected. An increase of the N-DMR induced by EGF was observed with low concentrations of adenoviral particles (i.e., MOI of about 1 to about 10) (Fig. 7A). At higher doses of virus (MOI of about 100 to about 1,500) a decrease in the signal induced by EGF was observed (see Fig. 7B). We were also able to observe a dose dependent response of EGF-induced N-DMR by adenoviral particles (see Fig. 7C).

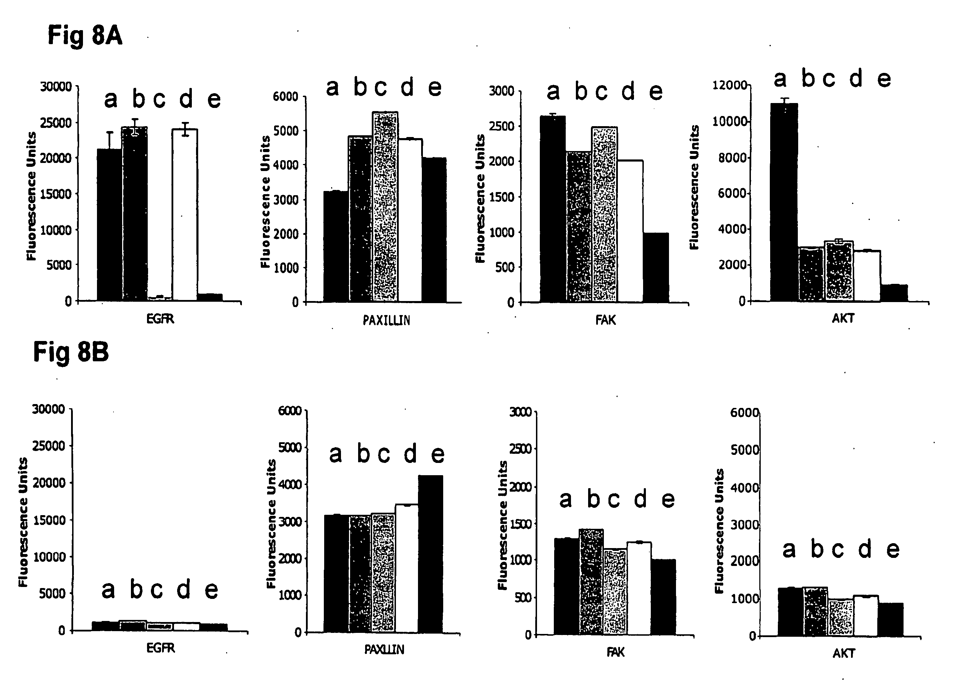

**Viral Hijacking of cell signaling analyzed with phosphoarray assay**. - To confirm our data and also to have a better understanding of the mechanism by which adenoviral particles could hijack the cell signaling, we analyzed the phosphorylation pattern of four proteins involved in diverse signaling pathway using a Mercator™ Phosphoarray assay system from Biosource. Thus, A431 cells were infected overnight in serum-free medium with various doses of Adenovirus-GFP (MOI of about 1, c, about 10, b or about 500, a). As a negative control A431 cells were treated only with serum-free medium (mock control bar "d"). At 20 hrs post infection, the infected and non-infected A431 cells were washed and then stimulated with 32 nM EGF for 30 min (Figure 8A), 25 nM epinephrine for 1hr (Figure 8B), or serum-free medium for 1 hr at ambient room temperature (column e in Figure 8A and 8B, cell non infected and untreated). Cells extracts were then prepared according to the recommended protocol and analyzed using Mercator™ phosphoarray assay. Fig. 8A shows the phosphoarray results for cell proteins EFGR, Paxillin, FAK, and AKT, that were stimulated with EGF. The letter labeled bars (a-e) represent: a = EFG/-adenovirus MOI 500, b = EFG/adenovirus MOI 10, c = EFG/ adenovirus MOI 1, d = EFG-mock, and e = untreated cells. Fig. 8B shows phosphoarray cell proteins EFGR, Paxillin, FAK, and AKT, that were stimulated with epinephrine. The letter labeled bars (a-e) are as above for Fig. 8A.

The phosphorylation pattern of EGFR, Paxillin, Fak and Akt in non-infected A431 cells, stimulated with EGF or Epinephrine, was first checked. As shown in the Fig. 8 (comparison of EGF- or epinephrine-mock control (d) and untreated cells (e)) in response to EGF stimulation, the phosphorylation levels of both EGFR and FAK increased significantly, whereas the phosphorylation level of paxillin was only mildly increased. These results suggest that EGFR signaling leads to cell detachment primarily through FAK, but not paxillin, consistent with literature reports (Lu, Z.; Jiang, G.; Blume-Jensen, P.; Hunter, T., Mol. Cell Biol., 2001, 21, 4016-4031). In response to epinephrine, the phosphorylation levels of both EGFR, Akt and FAK do not seem to be altered, while the phosphorylation level of paxillin decreases, leading to the increase in cell adhesion. These results confirm one hypothesis that the P-DMR in beta2AR optical signal is due to the increase in cell adhesion, but also lead to the identification of potential mechanism accounting for the increase in cell adhesion by the activation of beta2 adrenergic receptors. Fig. 9 shows a schematic of an example of signaling of cell migration. Thus for example a pathogen intrusion event, such as interaction of a pathogen with a cell's GPCR 9l0 or EFGR 920, can lead to, for example, pathway activation 950, pathway inhibition 960, pathway cleavage 970, or combinations thereof. Such pathway changes may further influence or cause changes in adhesion subunits alpha and beta 980 with respect to the extracellular matrix 990.

Fig. 9 summarizes events involved in cell migration and is consistent with other observations and measurements. The function of calpain is to digest the links between the actin cytoskeleton and focal adhesion proteins, such as Talin, paxillin, and focal adhesion kinase (FAK). The release of focal adhesion proteins from the complex, together with directional remodeling of cytoskeletal structure, helps facilitate migration. Calpain2 is thought to be a membrane bound protein that functions at the trailing edge of the migrating cell to cleave the integrins in response to growth factor receptor signals. Down regulation of calpain2 is achieved by protein kinase A (PKA) activated in $G_s$ pathway.

Additionally, we investigated the effect of adenoviral infection on the phosphorylation pattern in A431 cells stimulated with EGF or epinephrine (comparison of EGF- or epinephrine-mock control with EGF- or epinephrine-Adenovirus). First, we observed that regardless of the dose of viruses, the adenoviral infection did not induce phosphorylation of the 4 proteins studied (data not shown). Instead, in A431 cells stimulated with epinephrine, adenoviral infection resulted in a decrease of phosphorylated Paxillin. This result confirms that the adenoviral infection increased the P-DMR induced by epinephrine in A431 cells and also that the increase in adhesion is the major contributor to the P-DMR event mediated by epinephrine.

At low doses (MOI of from about 1 to about 10), adenoviral particles were able to increase the level of phosphorylated Paxillin and Fak mediated by EGF (comparison of EGF-ADENO1 and EGF-mock control). In contrast, at higher doses (e.g., MOI of about 500), adenoviral infection resulted in a decrease of activated Paxillin in A431 cells stimulated with EGF. This data confirms and explains the results obtained using MRCAT and shows an increase of cell detachment at low doses of virus (increase in N-DMR) and an increase of cell adhesion at higher doses of virus (decrease in N-DMR). Interestingly, a high dose of adenovirus (MOI of about 500) dramatically increased the activated Akt (involved in the survival pathway), in accord with the literature, whereas a low dose (E.G., MOI of about 1) of adenovirus completely abolished the autophosphorylation of EGFR in cells stimulated with EGF.

Fig. 10 shows a schematic of an example of G-protein-coupled-receptor, EGFR, and focal adhesion signaling, which shows the potential network interactions mediated through different classes of cellular targets.

Fig. 11 shows kinetic responses of HeLa cells to adenoviral infection. Hela cells were subjected to the HBSS buffer (1110) and two different concentrations of adenovirus MOI 12000 (1120) and MOI 3,000 (1130). The cell response was plotted as a function of time.

Fig. 12 shows modulation of the adenovirus-induced response in HeLa cells with several modulators. HeLa cells, pretreated for 1h with HBSS (positive control) (1210), 10 microM Latrunculin A (1220), 10 microM cytochalasin B (1230), or (D) 40 microM DIPC (1240), were infected with adenovirus (MOI 6,000).

Fig. 13 shows example results of DIPC inhibition of an adenoviral infection in HeLa cells. HeLa cells, pretreated for 1h with HBSS (no treatment), or 40 microM dynamin inhibitory peptide (DIPC)(treatment), were infected with increased doses of adenovirus (MOI 6000 to 1500). 24h after the infection, viral infection efficiency was checked by fluorescence microscopic observations (expression of GFP).

**Potential application to diagnostics for inflammatory diseases** Typical applications used in the area of diagnostic technologies in inflammatory diseases are based on, for example, chemokine detection, chemokine-receptor binding assays, enzymatic assays, and antibody recognition. With a rapidly-growing world market for anti-inflammatory agents topping $50 billion a year, research is also focused on the application of emerging technologies to innovate drug discovery in this field, for example, using two powerful discovery technologies: gene chips and proteomics. "Gene chips" allow the most important molecules in very complex disease processes to be identified as this technology monitors simultaneous changes in literally tens of thousands of genes concurrently. "Proteomics" is an advanced analytical method that measures minute changes in the expression patterns of proteins in cells and tissues and can also be used to recognize and understand how the regulation of cellular biochemistry is altered in disease.

An RWG biosensor utilizes the resonant coupling of light into a waveguide by means of a diffraction grating. There are many types of detection schemes, for example, wavelength and angular interrogation systems. In a wavelength interrogation system, polarized light, covering a range of incident wavelengths, is used to directly illuminate the waveguide; light at specific wavelengths is coupled into and propagates along the waveguide. The resonance wavelength at which a maximum in-coupling efficiency is achieved is a function of the local refractive index at or near the sensor surface. When a target molecule in a sample binds to a cellular target it triggers a dynamic relocation or redistribution of cellular contents within the bottom portion of the layer of the cell system or the biological system (i.e., within the detection zone or sensing volume of the optical biosensor), and is accompanied by a shift in the resonance wavelength. Although not limited by theory, the dynamic relocation or redistribution of cellular contents may be attributable to, for example, the dynamic relocation of any cellular targets, the change in the morphology (such as cell rounding or flattening, or cytoskeletal remodeling) of the cell system induced by the stimulation of the cell system with a ligand, or both.

An example of a commercial instrument embodying the resonance wavelength method is the Corning® Epic®

system (www.corning.com/lifesciences), which includes an RWG detector having, for example, a temperature-controlled environment and a liquid handling system. The detector system includes integrated fiber optics to measure the ligand-induced wavelength shift of the reflected light. A broadband light source, generated through a fiber optic and a collimating lens at nominally normal incidence through the bottom of the microplate, can be used to illuminate a small region of the grating surface. A detection fiber for recording the reflected light is bundled with the illumination fiber. A series of illumination/detection heads are arranged in a linear fashion, so that reflection spectra are collected from a subset of wells within the same column of, for example, a 384-well microplate simultaneously. The whole plate is scanned by the illumination/detection heads so that each sensor can be addressed multiple times, and each column is addressed in sequence. The wavelengths of the reflected light are collected and used for analysis. An optional temperature-controlling unit can minimize temperature fluctuation.

In an alternative angular interrogation system, a polarized light, covering a range of incident angles, is used to directly illuminate the waveguide; light at specific angles is coupled into and propagates along the waveguide. The resonance angle at which a maximum in-coupling efficiency is achieved is a function of the local refractive index at or near the sensor surface. When target molecules in a sample bind to a cellular target in a live-cell system and trigger a cellular response within the bottom portion of the layer of the cell system or the biological systems, the resonance angle shifts. Such a system is described in, for example, U.S. Patent Publication No. US-2004-0263841, U.S. Patent Publication No. US-2005-0099622, and U.S. Patent Publication No. US-2005-0236554.

For cell-based assays of the present disclosure, live-cells can be contacted with a suitable surface of a biosensor, for example, via culturing. The cell adhesion can be mediated through, for example, three types of contacts: focal contacts, close contacts, or extracellular matrix (ECM) contacts. Each type of contact has its own characteristic separation distance from the surface. As a result, cell plasma membranes are about 10 to about 100 nm away from the substrate surface, so that optical biosensors of relatively short penetration depths are still able to sense the bottom portion of the cells proximate to the biosensor surface. A phenomenon that is common to many stimuli-induced cell responses is dynamic relocation or rearrangement of certain cellular contents; some of which can occur within the bottom portion of cells proximate to the biosensor surface. Dynamic relocation or rearrangement of cellular contents can include, for example, changes in adhesion degree, membrane ruffling, recruiting intracellular proteins to activated receptors at or near a cell's surface, receptor endocytosis, and like phenomena. A change in cellular contents within the sensing volume leads to an alteration in local refractive index near the sensor surface, which manifests itself as an optical signal from the biosensor.

Based on the configuration of the biosensors used and the uniqueness of cell properties, the penetration depth of the $TM_0$ mode for Corning® Epic® RWG biosensor microplates is, for example, about 150 nm. Such relatively short penetration depth or sensing volume is common to most types of label-free optical biosensor technologies including conventional SPR and RWG, so that the disclosure is applicable to other optical biosensor-based cell sensing.

Theoretical analysis suggests that the detected signal, in terms of wavelength or angular shifts, is primarily sensitive to the vertical mass redistribution. Because of its dynamic nature, it is also referred to as a dynamic mass redistribution (DMR) signal. Beside the DMR signal, the biosensor is also capable of detecting horizontal (i.e., parallel to the sensor surface) redistribution of cellular contents. Theoretical analysis, based on the zigzag theory, shows that any changes in the shape of a resonant peak are mainly due to ligand-induced inhomogeneous redistribution of cellular contents parallel to the sensor surface (see Fang, Y., Ferrie, A.M., Fontaine, N.H., Mauro, J., and Balakrishnan, J. (2006) "Resonant Waveguide Grating Biosensor for Live Cell Sensing," Biophys. J., 91, 1925-1940). In addition, the DMR signal is a sum of all redistribution events within the sensing volume. This suggests that whole cell sensing with the biosensors of the disclosure is distinct from the aforementioned affinity-based assays, which directly measure the amount ofanalyte binding to the immobilized receptors.

Example 3

**Optical biosensor for monitoring the impact of viral infection on cell growth and cell signaling** In this example, cells in a culture medium are freshly mixed with a certain number of virus particles, and the resultant cell solution is placed onto each well of a 384-well Corning® Epic® biosensor microplate. After culturing, cells become adherent on the surface of each biosensor, and become infected by the virus. The optical biosensor output (e.g., shift in resonant wavelength or angles) is directly proportional to the cell density (i.e., confluency) as well as the adhesion degree. Thus, the impact of viral infection on the cell growth and adhesion can be directly assessed by measuring the changes in optical output before and after the cell attachment, and comparing with the changes in these control wells where the cells alone are placed into. Since the cells become infected during culturing, the incidence of viral infection and the impact of viral infection on cell signaling can also be assayed using panels of modulators or markers, by following the disclosed protocol (see for example in Example 2). Such an approach enables the detection of viral infection using the cell signaling network mapping approach, and also enables the examination of the impact of viral infection on the cell growth (i.e., proliferation) and the degree cell adhesion.

Example 4

**Electrical biosensor for viral detection and hijacking of cell signaling** The disclosure also provides methods which can be used in other optical biosensors, such as SPR, as well as other biosensor, such as an electric impedance-based biosensor, so that cells can attach and grow on these surfaces, and can also permit the attached cells to be assayed in accord with the present disclosure. Specifically, SPR uses a thin layer of gold film as a substrate. The gold surface can be modified such that cells can attach and grow on these surfaces. For example, the gold surface can be modified by passive immobilization of a thin layer of fibronectin or collagen, which can be optimized for cell attachment. To minimize the impact of coating on the biosensor sensitivity, low-density coating of biological material or nanopatterned biological material can also be prepared, for a related example see U.S. Patent No. 6,893,705. Cells cultured onto these surfaces can be used for assaying ligand-induced cellular activities of both adherent and weakly adherent cells. Biosensor surfaces having, for example, reactive species or bio-interacting molecules, can also be made on the gold substrate. These modified biosensor surfaces can also be applied to assay ligand-induced cellular activities of suspension cells.

Alternatively, electrical biosensors such as MDS Sciex CellKey system or Acea Biosciences RT-CES system can also be used to study the viral infection. Electrical biosensors consist of a substrate (e.g., plastic), an electrode, and a cell layer. In this electrical detection method, cells are cultured on small gold electrodes arrayed onto a substrate, and the system's electrical impedance is followed with time. The impedance is a measure of changes in the electrical conductivity of the cell layer. Typically, a small constant voltage at a fixed frequency or varied frequencies is applied to the electrode or electrode array, and the electrical current through the circuit is monitored over time. The CellKey system consists of an environmentally controlled impedance measurement system, a 96-well electrode-embedded microtiter plate, an onboard 96-well fluidics, and custom acquisition and analysis software. The cells are seeded in the culture wells; each well has an integrated electrode array. The system operates using a small-amplitude alternating voltage at 24 frequencies, from 1 KHz to 10 MHz. The resultant current is measured at an update rate of 2 sec. The system is thermally

regulated and experiments can be conducted between 28°C and 37°C. A 96-well head fluid delivery device handles fluid additions and exchanges onboard.

The RT-CES system is composed of four main components: electronic microtiter plates (E-Plate™), E-Plate station, electronic analyzer, and a monitoring system for data acquisition and display. The electronic analyzer sends and receives the electronic signals. The E-Plate station is placed inside a tissue culture incubator. The E-Plate station comes in three throughput varieties: a 16x station for running six 16-well E-Plates at a time, a single 96-well E-Plate station, and the Multi-E-Plate™ station, which can accommodate up to six 96-well E-Plates at a time. The cells are seeded in E-Plates, which are integrated with microelectronic sensor arrays. The system operates at a low-voltage (less than 20 mV) AC signal at multiple frequencies.

Following the protocol described in the present disclosure, a viral infection can be also detected and examined using these electrical biosensors. This is because both CellKey and RT-CES systems also provide an integrated cellular response, similar to the optical biosensors. Instead of dynamic mass redistribution (DMR) signals measured by optical biosensors, these electrical biosensors measure a bioimpedance signal. Using the cell-signaling mapping approach the present disclosure describes the impact of viral infection on cell-signaling and network interactions can be mapped out. The resulting pattern of the impact of viral infection on a panel of marker-induced bioimpedance signals can be used as a signature of the type of virus.

The disclosure has been described with reference to various specific embodiments and techniques. However, it should be understood that many variations and modifications are possible while remaining within the scope of the disclosure.

**Claims**

**1.** A label-free method to detect pathogen intrusion in a live-cell, the method comprising:

providing an evanescent wave biosensor having a live-cell immobilized on a surface of the biosensor;
detecting the dynamic mass redistribution (DMR) signal induced by a panel of markers on one or more cell-signal pathways , each marker comprising a molecule, a biomolecule or a biological that can modulate an activity of at least one cellular target, and result in a reliably detectable biosensor output as measured by the biosensor;
contacting the immobilized cell on the surface of the biosensor with a pathogen;
detecting a perturbation in the DMR signal induced by the panel of markers on the one or more cell-signal pathways;
and
equating the perturbation with: the extent of pathogen intrusion; the alteration in cellular activity attributable to pathogen intrusion; the cell's inflammatory response; or combinations thereof,
the biosensor output comprises a shift in resonant wavelength, a shift in resonant angle, or a change in peak width at half-maximum of the resonant peak.

2. The method of claim 1 further comprising contacting the immobilized cell on the surface of the biosensor with a prophylactic candidate or therapeutic candidate either before or after contacting the immobilized cell on the surface of the biosensor with a pathogen.

3. The method of claim 1 wherein
the evanescent biosensor includes a signal recognition element and a transducer element;
the pathogen comprises at least one of a virus, a bacteria, a prion, or combinations thereof;
the pathogen intrusion comprises a cell's response or an immune response to the pathogen;
the cell comprises a cell line, or a cell system;
the cell-signal pathway comprises at least one of a $Ca^{2+}$ pathway, a mitogen-activated protein kinase pathway, an adhesion pathway, a cAMP pathway, an apoptotic pathway, cell cycle pathway, or combinations thereof.

## Patentansprüche

1. Markierungsfreies Verfahren zur Detektion der Intrusion von Pathogen in eine lebende Zelle, wobei das Verfahren Folgendes umfasst:

Bereitstellen eines Evaneszent-Wellen-Biosensors, bei dem eine lebende Zelle an einer Oberfläche des Biosensors immobilisiert ist;
Detektieren des Signals der dynamischen Massenumverteilung (DMR), das durch eine Gruppe von Markern auf einem oder mehreren zellulären Signalwegen induziert wird, wobei jeder Marker ein Molekül, ein Biomolekül oder einen biologischen Stoff umfasst, das/der eine Aktivität von mindestens einem Zellziel modulieren und zu einem zuverlässig detektierbaren Ergebnis des Biosensors, wie mit dem Biosensor gemessen wurde, führen kann;
Inkontaktbringen der immobilisierten Zelle auf der Oberfläche des Biosensors mit einem Pathogen;
Detektieren einer Störung des DMR-Signals, das durch die Gruppe von Markern auf einem oder mehreren zellulären Signalwegen induziert wird;
und
Gleichsetzen der Störung mit: dem Ausmaß an Intrusion von Pathogen; der Veränderung der zellulären Aktivität, die auf die Intrusion von Pathogen zurückgeführt werden kann; der Entzündungsreaktion der Zelle; oder Kombinationen davon,
wobei das Ergebnis des Biosensors eine Verschiebung der Resonanzwellenlänge, eine Verschiebung des Resonanzwinkels oder eine Veränderung der Peak-Halbwertsbreite des Resonanzpeaks umfasst.

2. Verfahren nach Anspruch 1, das weiter das Inkontaktbringen der immobilisierten Zelle auf der Oberfläche des Biosensors mit einem prophylaktischen Kandidaten oder therapeutischen Kandidaten entweder vor oder nach dem Inkontaktbringen der immobilisierten Zelle auf der Oberfläche des Biosensors mit einem Pathogen umfasst.

3. Verfahren nach Anspruch 1, worin
der evaneszente Biosensor ein Signalerkennungselement und Signalwandlerelement einschließt;
das Pathogen mindestens eines von einem Virus, einem Bakterium, einem Prion oder Kombinationen davon umfasst;
die Intrusion von Pathogen eine Zellreaktion oder eine Immunantwort auf das Pathogen umfasst;
die Zelle eine Zelllinie oder ein Zellsystem umfasst; der zelluläre Signalweg mindestens einen von folgenden umfasst: einen $Ca^{2+}$-Weg, einen Weg der Mitogen-aktivierten Proteinkinasen, einen Adhäsionsweg, einen cAMP-Weg, einen apoptotischen Weg, Zellzyklusweg oder Kombinationen davon.

## Revendications

1. Procédé exempt de traceur pour détecter l'intrusion d'un agent pathogène dans une cellule vivante, le procédé comprenant :

la fourniture d'un biocapteur à onde évanescente ayant une cellule vivante immobilisée à la surface du biocapteur ;

la détection du signal de redistribution de masse dynamique (RMD) induit par un panel de marqueurs sur une ou plusieurs voies de signalisation cellulaire,

chaque marqueur comprenant une molécule, une biomolécule ou un agent biologique qui peut moduler une activité d'au moins une cible cellulaire et

conduire à une sortie du biocapteur détectable de manière fiable telle que mesurée par le biocapteur ;

la mise en contact de la cellule immobilisée à la surface du biocapteur avec un agent pathogène ;

la détection d'une perturbation du signal RMD induit par le panel de marqueurs sur la ou les voies de signalisation cellulaire ;

et

l'association de la perturbation à : l'étendue de l'intrusion de l'agent pathogène ; l'altération de l'activité cellulaire pouvant être attribuée à l'intrusion de l'agent pathogène ; la réponse inflammatoire de la cellule ; ou des combinaisons de celles-ci,

la sortie du biocapteur comprend un décalage de la longueur d'onde résonante, un décalage de l'angle résonant ou un changement de largeur du pic à mi-hauteur du pic résonant.

2. Procédé selon la revendication 1, comprenant en outre la mise en contact de la cellule immobilisée à la surface du biocapteur avec un candidat prophylactique ou un candidat thérapeutique avant ou après la mise en contact de la cellule immobilisée à la surface du biocapteur avec un agent pathogène.

3. Procédé selon la revendication 1, dans lequel le biocapteur évanescent inclut un élément de reconnaissance de signal et un élément transducteur ; l'agent pathogène comprend au moins un élément parmi un virus, une bactérie, un prion ou des combinaisons de ceux-ci ;

l'intrusion de l'agent pathogène comprend la réponse d'une cellule ou une réponse immunitaire à l'agent pathogéne ;

la cellule comprend une lignée cellulaire ou un système cellulaire ;

la voie de signalisation cellulaire comprend au moins une voie parmi une voie $Ca^{2+}$, une voie de protéine kinase activée par des agents mitogènes, une voie d'adhésion, une voie cAMP, une voie apoptotique, une voie de cycle cellulaire ou des combinaisons de celles-ci.

Fig 1

**Fig 2**

Fig 3

Fig 4A

Fig 4B

Fig 5B

Fig 5A

## Fig 6A

## Fig 6B

EP 2 064 542 B1

Fig 7A

Fig 7B

Fig 7C

EP 2 064 542 B1

Fig 8A

Fig 8B

Fig 9

## Fig 10

## Fig 11

## Fig 12

Fig 13

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20060223051 A
- US 20040263841 A
- US 20050099622 A
- US 20050236554 A
- US 2006013539 W
- WO 2006108183 A
- US 6893705 B


**Non-patent literature cited in the description**

- **Fang, Y. et al.** *J. Pharmacol. Tox. Methods,* 2007, vol. 55, 314-322
- **Fang, Y. et al.** *Anal. Chem.,* 2005, vol. 77, 5720-5725
- **Fang, Y. et al.** *FEBS Lett.,* 2005, vol. 579, 4175-4180
- **Giaever, I. ; Keese, C.R.** *Proc. Natl. Acad Sci. U.S.A.,* 1984, vol. 81, 3761
- **Solly, K. ; Wang, X. ; Xu, X. ; Strulovici, B ; Zheng, W.** *Assays Drug Dev. Technol.,* 2004, vol. 2, 363
- **Verdonk, E. ; Johnson, K. ; McGuinness, R ; Leung, G. ; Chen, Y.-W. ; Tang, H.R. ; Michelotti, J.M. ; Liu, V.F.** *Assays Drug Dev. Technol.,* 2006, vol. 4, 609
- **Lu, Z. ; Jiang, G. ; Blume-Jensen, P. ; Hunter, T.** *Mol. Cell Biol.,* 2001, vol. 21, 4016-4031
- **Fang, Y. ; Ferrie, A.M. ; Fontaine, N.H. ; Mauro, J. ; Balakrishnan, J.** Resonant Waveguide Grating Biosensor for Live Cell Sensing. *Biophys. J.,* 2006, vol. 91, 1925-1940